# EUROPEAN PATENT SPECIFICATION

(11) **EP 3 727 406 B1**
(45) Date of publication and mention of the grant of the patent: **16.04.2025**
(21) Application number: 18830784.7
(22) Date of filing: 18.12.2018
(51) Int. Cl.: A61K 35/74, A61K 35/747, A23L 33/135, C12N 1/20, C12R 1/25, A61P 1/00, A61P 25/18, A61P 25/22, A61P 25/24, A61P 25/28

(54) **PROBIOTICS FOR COGNITIVE AND MENTAL HEALTH**
PROBIOTIKA ZUR COGNITIVEN UND MENTALEN GESUNDHEIT
PROBIOTIQUES POUR LA SANTÉ COGNITIVE ET MENTALE

(30) Priority: 19.12.2017 WO PCT/CN2017/117125
(43) Date of publication of application: 28.10.2020
(73) Proprietor: International N&H Denmark ApS, 2800 Kongens Lyngby (DK)
(72) Inventor: STENMAN, Lotta, 02460 Kantvik (FI); LEHTINEN, Markus, 02460 Kantvik (FI); MAO, Yuejian, Shanghai, 200335 (CN)
(74) Representative: International N&H EMEA
(86) International application number: PCT/EP2018/085473
(87) International publication number: WO 2019/121669

(56) References cited:
- EP-A1- 2 937 424
- WO-A1-2019/121666
- HWAN L C: "Composition useful e.g. for mental- and physical stress relief, and improving immunity, comprises e.g. red ginseng powder, crystalline cellulose, glutamic acid, glycine, Chinese matrimony vine extract powder, and Angelica gigas powder", WPI / 2017 CLARIVATE ANALYTICS,, vol. 2017, no. 54, 6 July 2017 (2017-07-06), XP002788691
- FOSTER JANE A. ET AL: "Stress & the gut-brain axis: Regulation by the microbiome", NEUROBIOLOGY OF STRESS, vol. 7, 1 December 2017 (2017-12-01), pages 124 - 136, XP093044205, ISSN: 2352-2895, DOI: 10.1016/j.ynstr.2017.03.001
- LOTTA K STENMAN ET AL: "Strain-specific, stress-modulating effects of probiotics: a systematicscreening in a mouse model of chronic restraint stress", INTERNET CITATION, 23 August 2018 (2018-08-23), XP002790321, Retrieved from the Internet <URL:http://ilsi.eu/wp-content/uploads/sites/3/2018/09/8.-Poster_Patterson.pdf>
- FOSTER JANE A. ET AL: "Stress & the gut-brain axis: Regulation by the microbiome", NEUROBIOLOGY OF STRESS, vol. 7, 1 December 2017 (2017-12-01), pages 124 - 136, XP093044520, ISSN: 2352-2895, DOI: 10.1016/j.ynstr.2017.03.001
- STENMAN LOTTA K. ET AL: "Strain specific stress-modulating effects of candidate probiotics: A systematic screening in a mouse model of chronic restraint stress", BEHAVIOURAL BRAIN RESEARCH., vol. 379, 1 February 2020 (2020-02-01), NL, pages 112376, XP055920716, ISSN: 0166-4328, DOI: 10.1016/j.bbr.2019.112376
- DATABASE WPI Week 200966, Derwent World Patents Index; AN 2009-J08435, XP002788689
- DATABASE WPI Week 201572, Derwent World Patents Index; AN 2015-47636C, XP002788690
- DATABASE WPI Week 201754, Derwent World Patents Index; AN 2017-47942P, XP002788691
- PARK SUNMIN ET AL: "Black carrots fermented withLactobacillus plantarumorAspergillus oryzaeprevent cognitive dysfunction by improving hippocampal insulin signalling in amyloid-[beta] infused rats", JOURNAL OF FUNCTIONAL FOODS, ELSEVIER BV, NL, vol. 25, 30 June 2016 (2016-06-30), pages 354 - 366, XP029665310, ISSN: 1756-4646, DOI: 10.1016/J.JFF.2016.06.018
- DAVIS DANIEL J ET AL: "Microbial modulation of behavior and stress responses in zebrafish larvae", BEHAVIOURAL BRAIN RESEARCH, ELSEVIER, AMSTERDAM, NL, vol. 311, 20 May 2016 (2016-05-20), pages 219 - 227, XP029628109, ISSN: 0166-4328, DOI: 10.1016/J.BBR.2016.05.040
- LIU YEN-WENN ET AL: "Psychotropic effects ofLactobacillus plantarumPS128 in early life-stressed and naïve adult mice", BRAIN RESEARCH, ELSEVIER, AMSTERDAM, NL, vol. 1631, 24 November 2015 (2015-11-24), pages 1 - 12, XP029396287, ISSN: 0006-8993, DOI: 10.1016/J.BRAINRES.2015.11.018
- LIU WEI-HSIEN ET AL: "Alteration of behavior and monoamine levels attributable toLactobacillus plantarumPS128 in germ-free mice", BEHAVIOURAL BRAIN RESEARCH, ELSEVIER, AMSTERDAM, NL, vol. 298, 29 October 2015 (2015-10-29), pages 202 - 209, XP029343593, ISSN: 0166-4328, DOI: 10.1016/J.BBR.2015.10.046
- NIMGAMPALLE MALLIKARJUNA ET AL: "Anti-Alzheimer Properties of Probiotic, Lactobacillus plantarumMTCC 1325 in Alzheimer's Disease induced Albino Rats.", JOURNAL OF CLINICAL AND DIAGNOSTIC RESEARCH : JCDR AUG 2017, vol. 11, no. 8, August 2017 (2017-08-01), pages KC01 - KC05, XP009511037, ISSN: 2249-782X
- DAVIS DANIEL J ET AL: "Lactobacillus plantarum attenuates anxiety-related behavior and protects against stress-induced dysbiosis in adult zebrafish", SCIENTIFIC REPORTS, vol. 6, 19 September 2016 (2016-09-19), XP009511036

## Description

### FIELD OF THE INVENTION

This invention relates to novel bacteria of the species *Lactobacillus plantarum* and to compositions comprising novel *Lactobacillus plantarum strains.* In particular, this invention relates to compositions of novel *Lactobacillus plantarum* strains for use in preventing and/or treating mental illness being a mood disorder, an anxiety disorder and/or depression, symptoms affecting mental health and/or a gastrointestinal disorder, such as irritable bowel syndrome in a mammal, characterised, for example, by psychological symptoms of depression, anxiety, deficits in cognitive function and dementia. This invention also relates to methods and uses of compositions comprising novel *Lactobacillus plantarum* strains, including food products, dietary supplements, and pharmaceutically acceptable formulations/compositions.

### BACKGROUND

Mental health is related to emotional, psychological, physical and social well-being. Our mental health status determines how we handle stress. A mental illness can be defined as a health condition that changes a person's thinking, feelings, or behaviour (or all three) and that causes the person distress and problems functioning in social, work or family activities. Mental illness encompasses a wide range of disorders related to anxiety, mood, psychosis, eating behaviour, impulse control and addiction, personality, sociability, dissociation, obsessive-compulsive and post-traumatic stress. Each illness alters a person's thoughts, feelings, and/or behaviours in distinct ways. Disorders such as Parkinson's disease, epilepsy and multiple sclerosis are brain disorders but they are considered neurological diseases rather than mental illness. Interestingly, the lines between mental illness and neurological diseases, including memory disorders such as mild cognitive impairment, dementia and Alzheimer's disease, are not clearly defined and increasing evidence now suggests that mental illness is associated with changes in the brain's structure, chemistry and function which could underlie the development of neurological disorders. For example, the link between neurocognitive deficits and mood disorders is well established such that in major depression, cognitive impairment can mimic that observed in dementia (Rabins et al. Br J Psychiatry 1984; 144: 488-92).

Furthermore, untreated chronic stress can result in serious health conditions such as anxiety, muscle pain, high blood pressure and a weakened immune system. Research shows that stress can contribute to the development of major illnesses, such as heart disease, depression and obesity. Symptoms of acute and chronic stress can manifest in the gastrointestinal tract, causing short- and long-term effects on the functions of the gastrointestinal tract, respectively. Exposure to stress results in alterations within the gut-brain axis, ultimately leading to the development of a broad array of gastrointestinal disorders including inflammatory bowel disease (IBD), irritable bowel syndrome (IBS) and other functional gastrointestinal diseases, food antigen-related adverse responses, peptic ulcers and gastroesophageal reflux disease (GERD). The major effects of stress on gut physiology include: 1) alterations in gastrointestinal motility; 2) increase in visceral perception; 3) changes in gastrointestinal secretion; 4) increase in intestinal permeability; 5) negative effects on regenerative capacity of gastrointestinal mucosa and mucosal blood flow; and 6) alteration in gut microbial composition (Konturek et al. J. Physiol Pharmacol. 2011; 62(6):591-9).

With respect to mental illness and associated neurocognitive decline and neurological disorders, there is now a clear emphasis on strategies to achieve positive mental and cognitive health for a full and healthy life. There is an increase in demand for nutritional therapies to achieve positive mental health, with no side effects. Current medication to treat mental illnesses symptoms affecting mental health have many negative side effects such as nausea, increased appetite and weight gain, fatigue and gastrointestinal symptoms. Dietary supplements may represent an attractive means of achieving positive mental health and preventing symptoms of mental illness and related conditions from developing.

The gut-brain axis describes the bidirectional communication that exists between the brain and the gut and the microbiota-gut-brain axis supports the role of the gut microbiome in this communication system. As outlined above, mental illness and symptoms affecting mental health are comorbid with gastrointestinal disorders whereby emotional and routine daily life stress can disrupt digestive function and vice versa. Increasing evidence indicates that the gut microbiota exerts a profound influence on brain physiology, psychological responses and ultimately behaviour (Dinan et al. J. Psychiatr Res. 2015; 63: 1-9). Emerging evidence suggests that probiotics can influence central nervous system function and regulate mood, psychological symptoms such as anxiety and depression and stress-related changes in physiology, behaviour and brain function.

EP2937424 discloses compositions comprising the lactic acid bacterium Lactobacillus plantarum PS128 deposited under DSMZ Accession No. DSM 28632 for prophylactic and treatment of stress-induced disorders selected from anxiety, depression, and irritable bowel syndrome.

### OBJECT OF INVENTION

It is an object of the present invention to provide means for preventing and/or treating a mental illness of a mood disorder, an anxiety disorder and/or depression, and/or a symptom affecting mental health being anxiety, mood swings and/or depression, and/or a gastrointestinal disorder, such as irritable bowel syndrome. It is therefore an object of the invention to provide means by which an individual's mental health can be promoted, maintained, and/or restored.

### SUMMARY OF THE INVENTION

The present invention is

### DESCRIPTION OF DRAWINGS

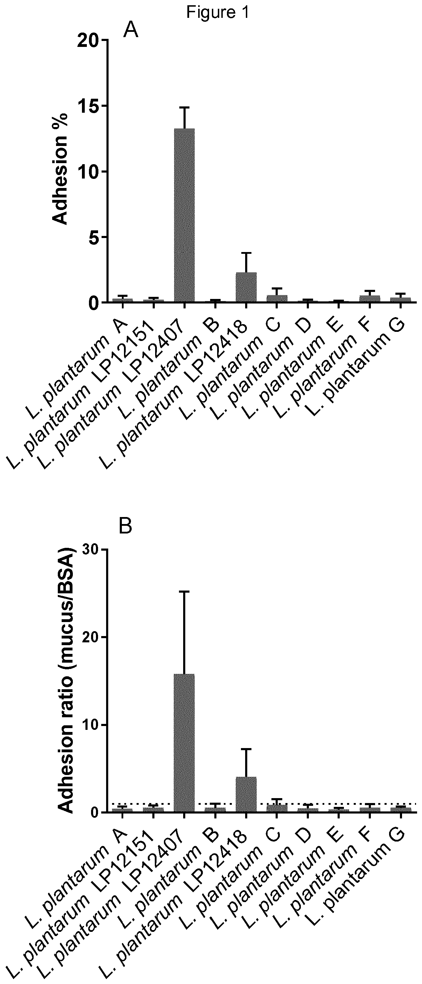

Figure 1. Determination of bacterial adhesion to intestinal mucus. Intestinal colonic mucus samples were collected from patients with colorectal cancer and the adhesion of ten different probiotic bacteria to human intestinal mucus was identified in an *in vitro* bacterial adhesion assay. Adhesion % (A) was determined as follows: (Fluorescence after adhesion - Blank) / (Maximum fluorescence - Blank) * 100. HEPES-Hank's buffer served as the blank. Adhesion ratio (B) was determined as: Adhesion to mucus / Adhesion to BSA. Values above 1 (dotted line) indicate higher affinity to mucus than BSA, which indicates mucus-specific binding capabilities. Data are shown as the mean +/- SD of data from five individual mucus samples.

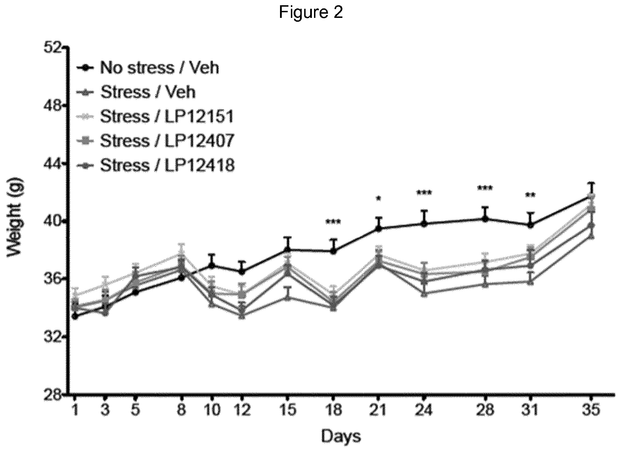

Figure 2. Effect of 3 weeks of chronic stress on body weight in mice, in groups treated with vehicle alone or a selected bacterial strain. N=12 for all groups except for stressed/vehicle group where N=11 and stressed/LP12418 group where N=11. Statistical Analyses: Two-way ANOVA; Weight (time): F_{(12,636)}=30.38, p < 0.0001; Weight (treatment): F_{(4,636)}=6.54, p < 0.0001

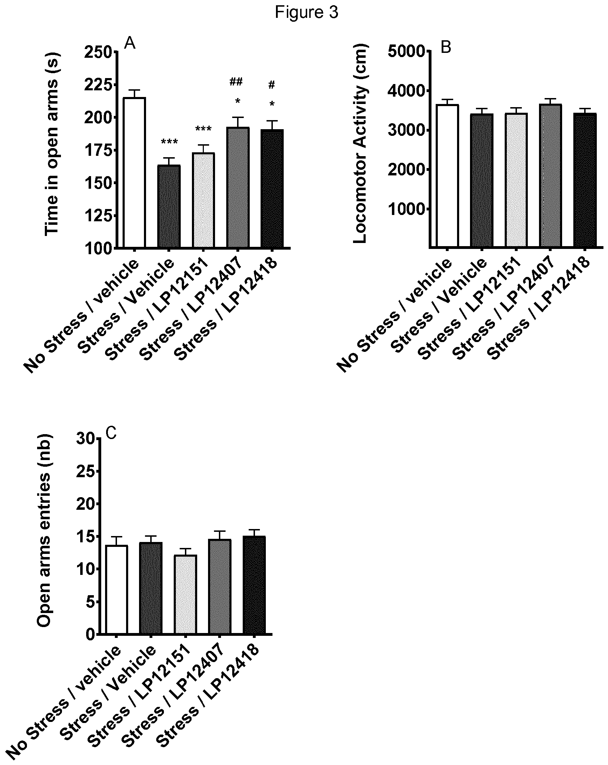

Figure 3. Effect of treatment with selected bacterial strains on chronic stress-induced anxiety in mice in the elevated plus maze procedure. Three parameters were measured; time in open arms (A), locomotor activity (B), and open arm entries (C). N=12 for all groups except for stressed/vehicle group where N=11 and stressed/LP12418 group where N=11. Statistical Analyses: One-way ANOVA; Time in open arms: F_{(4,57)}= 10.68, p < 0.0001; Locomotor activity: F_{(4,57)}= 1.057, p > 0.05; Open arm entries: F_{(4,57)}= 1.074, p > 0.05; Pairwise comparisons: * p < 0.05, *** p < 0.001 vs the non-stress/vehicle group, # p < 0.05, ## p < 0.01 vs chronic stress/vehicle group (Dunnett's test)

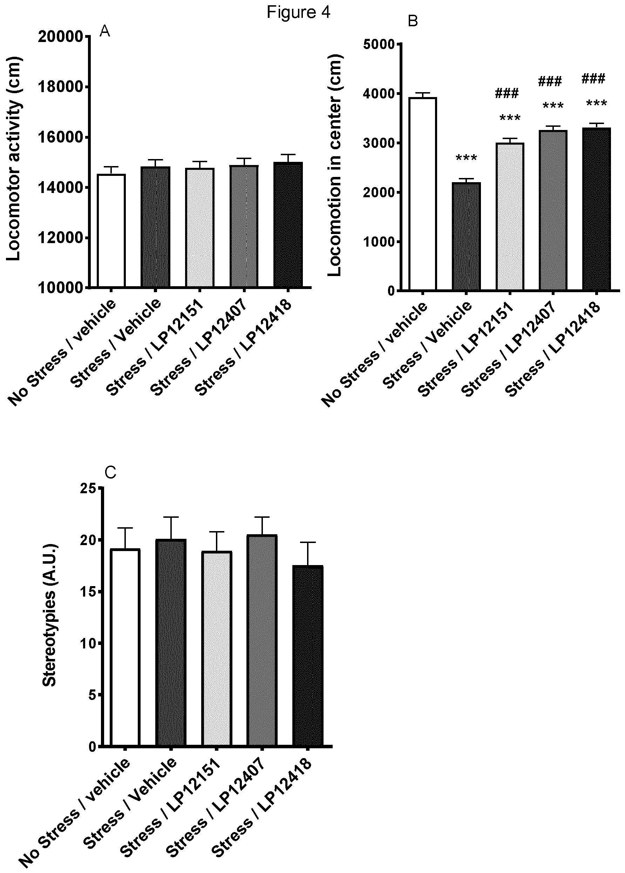

Figure 4. Effect of treatment with selected bacterial strains on chronic stress-induced anxiety in mice in the open field procedure. Three parameters were measured; locomotor activity (A), locomotion in the centre of the area (B), and rearing/grooming behaviour (stereotypies C). For stereotypies, data were represented as arbitrary units (A.U.) corresponding to the number of rearings plus the number of groomings during 10 minutes of open field procedure. N=12 for all groups except for stressed/vehicle group where N=11 and stressed/LP12418 group where N=11. Statistical analyses: One-way ANOVA; Locomotor activity: F_{(4,57)}= 0.4278, p > 0.05; Locomotion in the centre: F_{(4,57)}= 58.20, p < 0.0001; Stereotypes: F_{(4,57)}= 0.3330, p > 0.05; Pairwise comparisons: *** p < 0.001 vs. the non-stress/vehicle group, ### p < 0.001 vs the chronic stress/vehicle group (Dunnett's test)

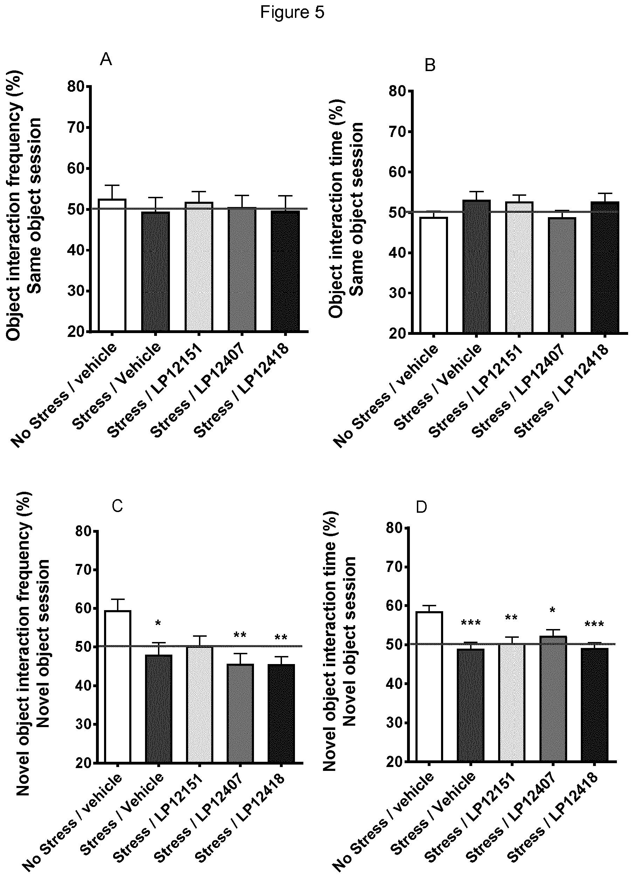

Figure 5. Effect of treatment with selected bacterial strains on chronic stress-induced recognition memory deficits in mice. Parameters measured included object interaction frequency (A, C) and object interaction time (B, D), for same object recognition (A, B) and novel object recognition (C, D). N=12 for all groups except for stressed/vehicle group where N=11 and stressed/LP12418 group where N=11. Statistical analyses: One-way ANOVA; Day 2 Same object frequency: F_{(4,57)}= 0.1884, p > 0.05; Day 2 Same object time: F_{(4,57)}= 1.627, p > 0.05; Day 3 Novel object frequency: F_{(4,56)}= 4.715, p = 0.0025; Day 3 Novel object time: F_{(4,56)}= 6.839, p = 0.0002; Pairwise comparisons: * p < 0.05, ** p < 0.01, *** p < 0.001 vs. the non-stress/vehicle group (Dunnett's test)

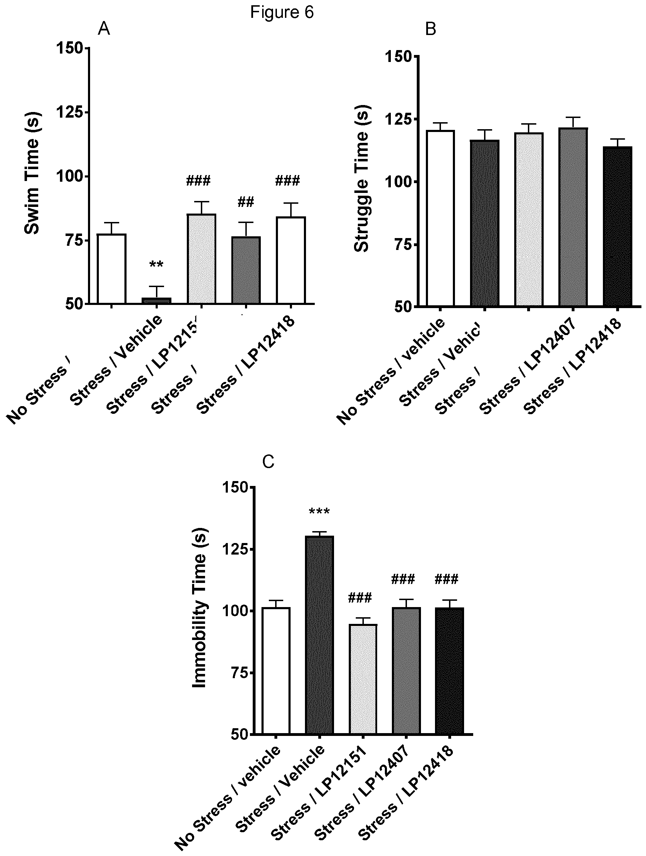

Figure 6. Effect of treatment with selected bacterial strains on chronic stress-induced behavioural despair in mice as measured in the forced swim test. Three parameters were measured; swim time (A), struggle time (B) and immobility time (C). N=12 for all groups except for stressed/vehicle group where N=11 and stressed/LP12418 group where N=11. Statistical analyses: One-way ANOVA; Swim time: F_{(4,57)}= 7.596, p < 0.0001; Struggle time: F_{(4,57)}= 0.8225, p > 0.05; Immobility time: F_{(4,57)}= 28.10, p < 0.0001; Pairwise comparisons: ** p < 0.01, *** p < 0.001 vs the non-stressed/vehicle group, ## p < 0.01, ### p < 0.001 vs the chronic stress/vehicle group (Dunnett's test)

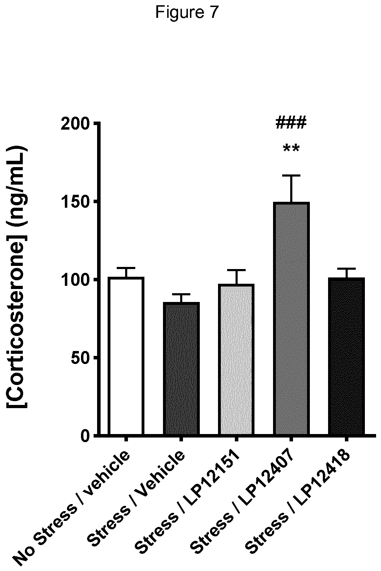

Figure 7. Effect of chronic stress and treatment with selected bacterial strains on plasma corticosterone concentrations. N=12 for all groups except for stressed/vehicle group where N=11 and stressed/LP12418 group where N=11. Statistical analyses: One-way ANOVA; Corticosterone concentration: F_{(4,57)}= 6.618, p = 0.0002. Pairwise comparisons ** p < 0.01 vs the non-stressed/vehicle group, ### p < 0.001 vs the chronic stress/vehicle group (Dunnett's test)

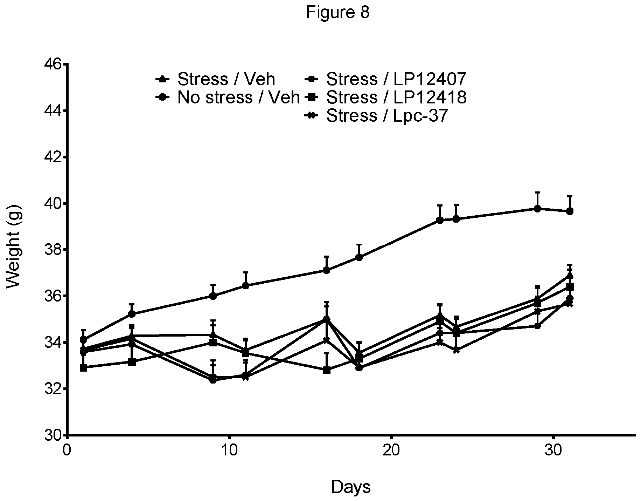

Figure 8. Effect of 3 weeks of chronic stress on body weight in mice, in groups treated with vehicle alone or a selected bacterial strain. N=10-18 for all groups. Statistical Analyses: Two-way ANOVA; Weight (time): F_{(9,643)}= 17.46, p < 0.0001; Weight (treatment): F_{(4,643)}= 74.39, p < 0.0001

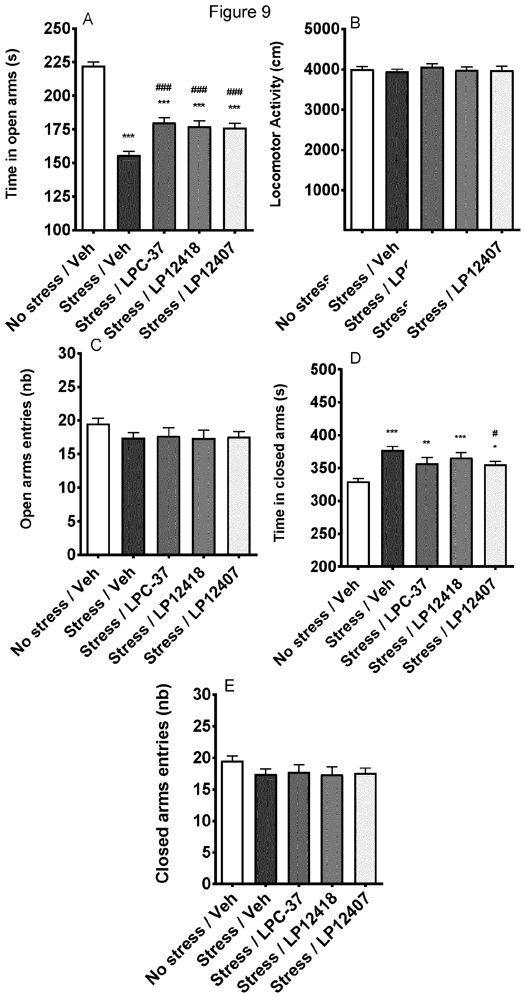

Figure 9. Effect of treatment with selected bacterial strains on chronic stress-induced anxiety in mice. N=10-18 for all groups. Statistical Analyses: One-way ANOVA; Time in open arms (A): F_{(4,67)}= 78.92, p < 0.0001; Locomotor activity (B): F_{(4,67)}= 0.3718, p > 0.05; Open arm entries (C): F_{(4,67)}= 1.19, p > 0.05; Time in closed arms (D): F_{(4,67)} = 10.85, p < 0.0001; Closed arm entries (E): F_{(4,67)} = 1.19, p > 0.05; Pairwise comparisons: * p < 0.05, ** p < 0.01, *** p < 0.0001 vs the non-stress/vehicle group, # p < 0.05, ### p < 0.0001 vs. the chronic stress/vehicle group (Dunnett's test)

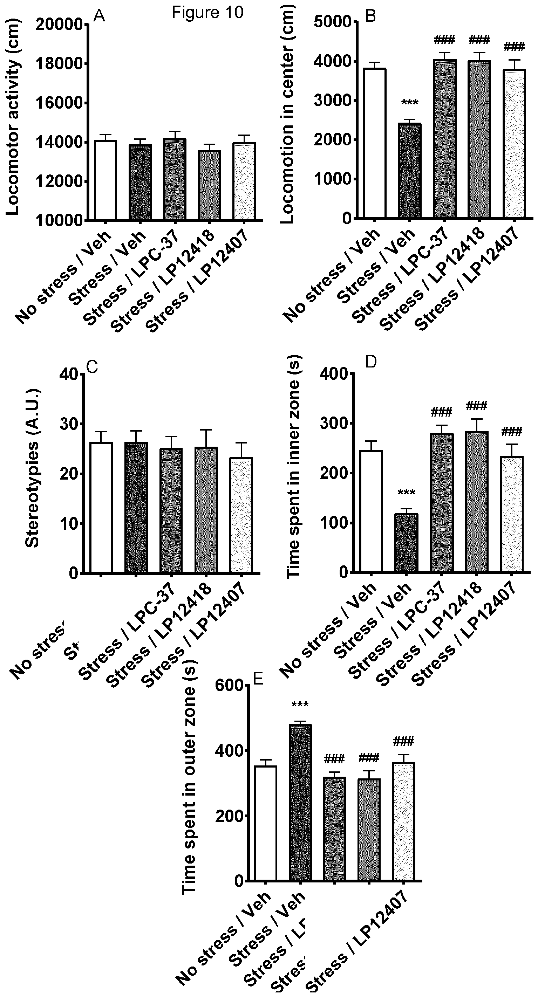

Figure 10. Effect of treatment with selected bacterial strains on chronic stress-induced anxiety in mice in the open field procedure. Five parameters were measured; locomotor activity (A), locomotion in the centre of the arena (B), rearing/grooming behaviour (stereotypies C), time spent in inner zone(s) (D), and time spent in outer zone(s) (E). For stereotypies, data were represented as arbitrary unit (A.U.) corresponding to the number of rearings plus the number of groomings during 10 minutes of open field procedure. N=10-18 for all groups. Statistical analyses: One-way ANOVA; Locomotor activity: F_{(4,67)}= 0.4977, p > 0.05; Locomotion in the centre: F_{(4,67)}= 21.5, p < 0.0001; Stereotypes: F_{(4.67)}= 0.2328, p > 0.05; Time spent in inner zone: F_{(4.67)}= 16.42, p < 0.0001; Time spent in outer zone: F_{(4.67)}= 16.42, p < 0.0001; Pairwise comparisons: *** p < 0.001 vs. the non-stress/vehicle group, ### p < 0.001 vs the chronic stress/vehicle group (Dunnett's test)

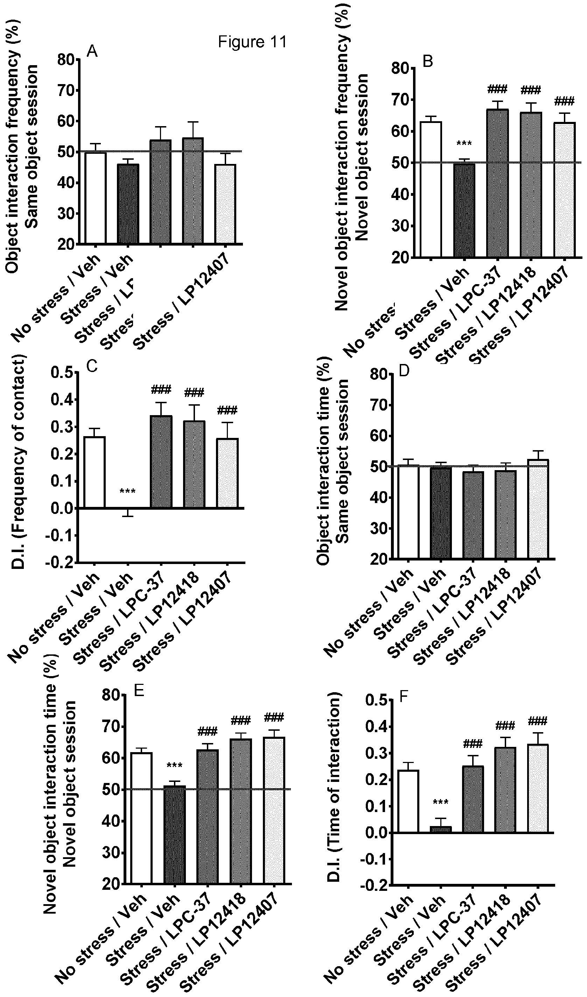

Figure 11. Effect of treatment with selected bacterial strains on chronic stress-induced recognition memory deficits in mice. Parameters measured included object interaction frequency (A, B) and object interaction time (D, E), for same object recognition (A, D) and novel object recognition (B, E). The discrimination index for the novel object (DI) as the difference of interaction frequency (C) and interaction time (F) mice spent investigating between the novel and the familiar object divided by the total frequency and time exploring both objects. [Discrimination Index, DI = (Novel Object Exploration frequency/time - Familiar Object Exploration frequency/time)/(Novel Object Exploration frequency/time + Familiar Object Exploration frequency/time)]. N=10-18 for all groups. Statistical analyses: One-way ANOVA; Day 2 Same object frequency: F_{(4,59)}= 1.54, p > 0.05; Day 2 Same object time: F_{(4,59)}= 0.5006, p > 0.05; Day 3 Novel object frequency: F_{(4,59)}= 14.18, p < 0.001; Day 3 Novel object time: F_{(4,59)}= 15.02, p < 0.001; Discrimination index frequency: F_{(4,59)}= 14.18, p < 0.0001, Discrimination index time: F_{(4,59)}= 15.02, p < 0.0001; Pairwise comparisons: *** p < 0.001 vs. the non-stress/vehicle group, ### p < 0.001 vs the chronic stress/vehicle group (Dunnett's test)

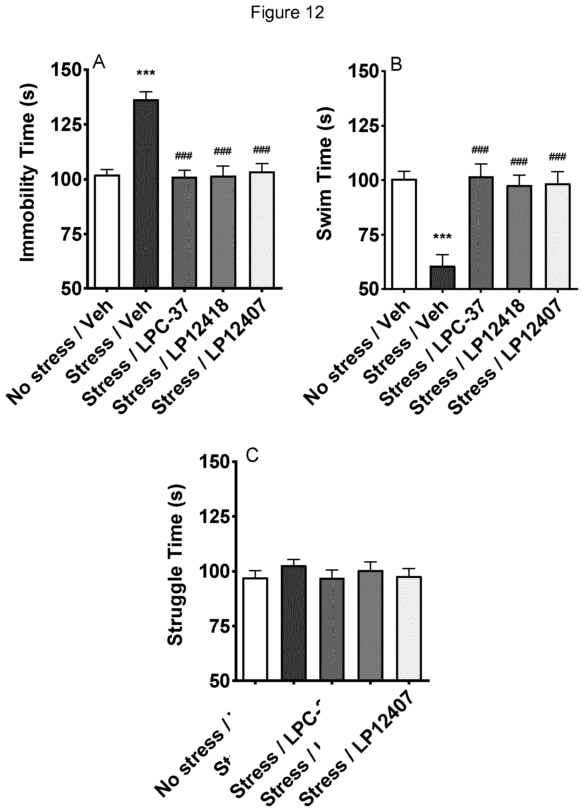

Figure 12. Effect of treatment with selected bacterial strains on chronic stress-induced behavioural despair in mice as measured in the forced swim test. Three parameters were measured; immobility time (A), swim time (B) and struggle time (C). N=10-18 for all groups. Statistical analyses: One-way ANOVA; Immobility time: F_{(4,59)}= 26.82, p < 0.0001; Swim time: F_{(4,59)}= 15.97, p < 0.0001; Struggle time: F_{(4,59)}= 0.7128, p > 0.05; Pairwise comparisons: *** p < 0.0001 vs the non-stressed/vehicle group, ### p < 0.0001 vs the chronic stress/vehicle group (Dunnett's test)

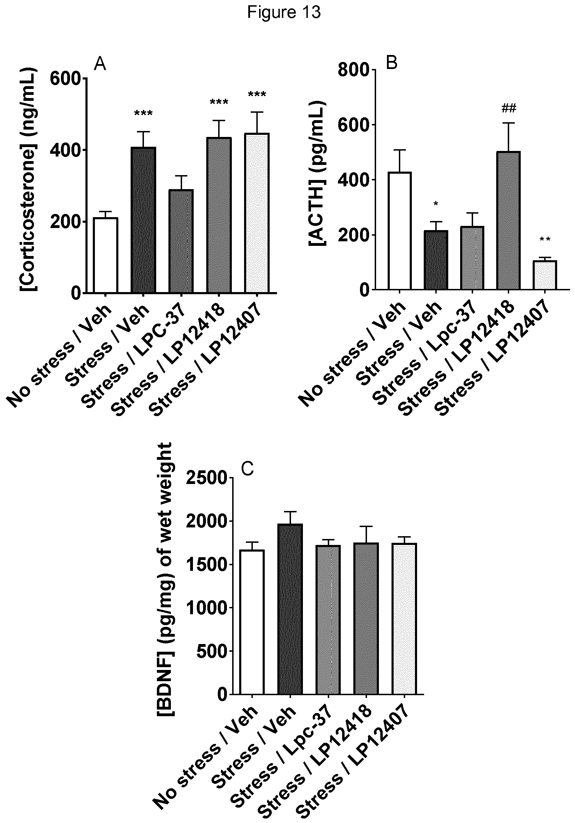

Figure 13. Effect of chronic stress and treatment with selected bacterial strains on blood plasma corticosterone (A) and adrenocorticotrophic hormone (ACTH) concentration (B) and hippocampus brain-derived neurotrophic factor (BDNF) concentration (C). N=8-18 for all groups. Statistical analyses: One-way ANOVA; Corticosterone concentration: F_{(4,67)}= 7.592, p < 0.001; ACTH concentration: F_{(4,67)}= 5.75, p < 0.001; BDNF concentration: F_{(4,67)}= 1.23, p > 0.05; Pairwise comparisons: * p < 0.05, ** p < 0.01, *** p < 0.001 vs. the non-stressed/vehicle group, ## p < 0.01 vs. the chronic stress/vehicle group (Dunnett's test)

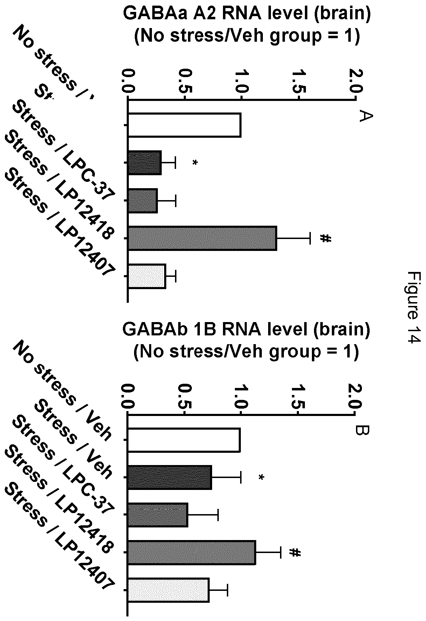

Figure 14: Effect of treatment with selected bacterial strains on chronic stress-induced expression fold change of GABA_{Aα2} (A) and GABA_{B1β} (B) receptor genes in the cortex. N=6 for all groups. The 2-DDCT method was used for differential expression analyses. Non-parametric tests were selected due to the low number of samples and Shapiro wilk normality test showed a p value < 0.05. * p < 0.05 vs. the non-stressed/ vehicle group, # p < 0.05 vs. the chronic stress/ vehicle group (Kruskal Wallis test and Nemenyi post-hoc test)

### DETAILED DESCRIPTION OF INVENTION

### Bacteria and Deposit

The bacteria used in aspects of the invention are bacteria of the species *Lactobacillus plantarum.* Novel *Lactobacillus plantarum* strains of the invention are:
a. *Lactobacillus plantarum* strain LP12418 deposited with the DSMZ (Leibniz-Institut DSMZ-Deutsche Sammlung von Mikroorganismen und Zellkulturen GmbH, Inhoffenstrasse 7B, D-38124 Braunschweig - Germany) under deposit number DSM 32655 on September 27,2017; and
*b. Lactobacillus plantarum* strain LP12407 deposited with the DSMZ (Leibniz-Institut DSMZ-Deutsche Sammlung von Mikroorganismen und Zellkulturen GmbH, Inhoffenstrasse 7B, D-38124 Braunschweig - Germany) under deposit number DSM 32654 on September 27, 2017.

Copies of the DSMZ deposit forms for LP12418 and LP12407 giving relevant information on the characteristics of the strains, are incorporated herein (LP12418 is referred to as DGCC12418 and LP12407 is referred to as DGCC12407).

The compositions of the invention include one or both of the above strains, and may, in addition, comprise *Lactobacillus paracasei* strain Lpc-37, also known as DGCC4981 or Lbc81. Strain Lpc-37 is registered at the ATCC under deposit number PTA 4798 and at the DSMZ (Leibniz-Institut DSMZ-Deutsche Sammlung von Mikroorganismen und Zellkulturen GmbH, Inhoffenstrasse 7B, D-38124 Braunschweig - Germany) under deposit number DSM32661 on October 5, 2017. A copy of the DSMZ deposit form for Lpc-37 giving relevant information on the characteristics of the strain, is incorporated herein.

Thus, the compositions of the invention comprise LP12418 or LP12407, or a combination of LP12418 and LP12406, or a combination of LP12418 and Lpc-37, or a combination of LP12407 and Lpc-37, or a combination of LP12418, LP12407 and Lpc-37.

The novel *Lactobacillus plantarum strains of the invention* may also be used in combination with one or more other bacterial strains which have the ability to exert positive health benefits on a host to which they are administered. Preferably, the other bacterial strains, when used in aspects of the invention, are suitable for human and/or animal consumption. A skilled person will be readily aware of specific bacterial strains which are used in the food and/or agricultural industries and which are generally considered suitable for human and/or animal consumption.

The *Lactobacillus plantarum* strains of the invention may be used in any form (for example viable, dormant, inactivated or dead bacteria) provided that the bacteria remain capable of exerting the effects described herein. Preferably, the *Lactobacillus plantarum* strains used in aspects of the invention are viable.

LP12418, LP12407 and Lpc-37 are probiotic bacteria. The term "probiotic bacteria" is defined as covering any non-pathogenic bacteria which, when administered live in adequate amounts to a host, confer a health benefit on that host. For classification as a "probiotic", the bacteria must survive passage through the upper part of the digestive tract of the host. They are non-pathogenic, non-toxic and exercise their beneficial effect on health on the one hand via ecological interactions with the resident flora in the digestive tract, and on the other hand via their ability to influence the host physiology and immune system in a positive manner. Probiotic bacteria, when administered to a host in sufficient number, have the ability to progress through the intestine, maintaining viability, exerting their primary effects in the lumen and/or the wall of the host's gastrointestinal tract. They then transiently form part of the resident flora and this colonisation (or transient colonisation) allows the probiotic bacteria to exercise a beneficial effect, such as the repression of potentially pathogenic microorganisms present in the flora and interactions with the host in the intestine including the immune system.

Optionally the novel *Lactobacillus plantarum* strains of the invention are used in combination with other probiotic bacteria.

### Compositions

The term "composition" is used in the broad sense to mean the manner in which something is composed, i.e. its general makeup.

In one aspect of the invention, the compositions may consist essentially of a single strain of *Lactobacillus plantarum* bacteria (e.g. LP12418 or LP12407).

Alternatively, the compositions may comprise a combination of LP12418 and LP12407.

The compositions of the invention may additionally comprise other components, such as other bacterial strains (e.g. Lpc-37), biological and chemical components, active ingredients, metabolites, nutrients, fibres, prebiotics, etc.

While it is not a requirement that the compositions of the invention comprise any support, diluent or excipient, such a support, diluent or excipient may be added and used in a manner which is familiar to those skilled in the art. Examples of suitable excipients include, but are not limited to, microcrystalline cellulose, rice maltodextrin, silicone dioxide, and magnesium stearate. The compositions of the invention may also comprise cryoprotectant components (for example, glucose, sucrose, lactose, trehalose, sodium ascorbate and/or other suitable cryoprotectants).

The terms "composition" and "formulation" may be used interchangeably.

Compositions used in aspects of the invention may take the form of solid, solution or suspension preparations. Examples of solid preparations include, but are not limited to: tablets, pills, capsules, granules and powders which may be wettable, spray-dried or freeze dried/lyophilized. The compositions may contain flavouring or colouring agents. The compositions may be formulated for immediate-, delayed-, modified-, sustained-, pulsed- or controlled-release applications.

By way of example, if the compositions of the present invention are used in a tablet form, the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of other acceptable carriers for use in preparing compositions include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, hydroxymethylceilulose, polyvinylpyrrolidone, and the like.

For aqueous suspensions and/or elixirs, the compositions of the present invention may be combined with various sweetening or flavouring agents, colouring matter or dyes, with emulsifying and/or suspending agents and with diluents such as water, propylene glycol and glycerin, and combinations thereof.

Specific non-limiting examples of compositions which can be used in aspects of the invention are set out below for illustrative purposes. These include, but are not limited to food products, functional foods, dietary supplements, pharmaceutical compositions and medicaments.

### Dietary Supplements

The compositions of the invention may take the form of dietary supplements or may themselves be used in combination with dietary supplements, also referred to herein as food supplements.

The term "dietary supplement" as used herein refers to a product intended for ingestion that contains a "dietary ingredient" intended to add nutritional value or health benefits to (supplement) the diet. A "dietary ingredient" may include (but is not limited to) one, or any combination, of the following substances: bacteria, a probiotic (e.g. probiotic bacteria), a vitamin, a mineral, a herb or other botanical, an amino acid, a dietary substance for use by people to supplement the diet by increasing the total dietary intake, a concentrate, metabolite, constituent, or extract.

Dietary supplements may be found in many forms such as tablets, capsules, soft gels, gel caps, liquids, or powders. Some dietary supplements can help ensure an adequate dietary intake of essential nutrients; others may help reduce risk of disease.

### Food products

The compositions of the invention may take the form of a food product. Here, the term "food" is used in a broad sense and covers food and drink for humans as well as food and drink for animals (i.e. a feed). Preferably, the food product is suitable for, and designed for, human consumption.

The food may be in the form of a liquid, solid or suspension, depending on the use and/or the mode of application and/or the mode of administration.

When in the form of a food product, the composition may comprise or be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the compositions of the invention may take the form of one of the following:
A fruit juice; a beverage comprising whey protein: a health or herbal tea, a cocoa drink, a milk drink, a lactic acid bacteria drink, a yoghurt and/or a drinking yoghurt, a cheese, an ice cream, a water ice, a desserts, a confectionery, a biscuit, a cake, cake mix or cake filling, a snack food, a fruit filling, a cake or doughnut icing, an instant bakery filling cream, a filling for cookies, a ready-to-use bakery filling, a reduced calorie filling, an adult nutritional beverage, an acidified soy/juice beverage, a nutritional or health bar, a beverage powder, a calcium fortified soy milk, or a calcium fortified coffee beverage.

Optionally, where the product is a food product, the *Lactobacillus plantarum* should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food ingredients

Compositions of the present invention may take the form of a food ingredient and/or feed ingredient.

As used herein the term "food ingredient" or "feed ingredient" includes a composition which is or can be added to functional foods or foodstuffs as a nutritional and/or health supplement for humans and animals.

The food ingredient may be in the form of a liquid, suspension or solid, depending on the use and/or the mode of application and/or the mode of administration.

### Functional Foods

Compositions of the invention may take the form of functional foods.

As used herein, the term "functional food" means food which is capable of providing not only a nutritional effect, but is also capable of delivering a further beneficial effect to the consumer.

Accordingly, functional foods are ordinary foods that have components or ingredients (such as those described herein) incorporated into them that impart to the food a specific function - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Medical Foods

Compositions of the present invention may take the form of medical foods.

By "medical food" it is meant a food which is formulated to be consumed or administered with or without the supervision of a physician and which is intended for a specific dietary management or condition for which distinctive nutritional requirements, based on recognized scientific principles, are established by medical evaluation.

### Pharmaceutical compositions

Compositions of the invention may be used as - or in the preparation of -pharmaceuticals. Here, the term "pharmaceutical" is used in a broad sense - and covers pharmaceuticals for humans as well as pharmaceuticals for animals (i.e. veterinary applications). In a preferred aspect, the pharmaceutical is for human use.

The pharmaceutical can be for therapeutic purposes - which may be curative or palliative or preventative in nature.

A pharmaceutical may be in the form of a compressed tablet, tablet, capsule, ointment, suppository or drinkable solution.

When used as - or in the preparation of - a pharmaceutical, the compositions of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a liquid or as a solid - depending on the use and/or the mode of application and/or the mode of administration.

The *Lactobacillus plantarum* strains used in the present invention may constitute the pharmaceutically active ingredient or ingredients. In certain embodiments, *Lactobacillus plantarum* LP12407 and/or LP12418 constitutes the sole active ingredient or ingredients. Alternatively, LP12407 and/or LP12418 may be present with one or more other pharmaceutically active ingredients.

### Medicaments

Compositions of the invention may take the form of medicaments.

The term "medicament" as used herein encompasses medicaments for both human and animal usage in human and veterinary medicine. In addition, the term "medicament" as used herein means any substance which provides a therapeutic, preventative and/or beneficial effect. The term "medicament" as used herein is not necessarily limited to substances which need Marketing Approval, but may include substances which can be used in cosmetics, nutraceuticals, food (including feeds and beverages for example), probiotic cultures, and natural remedies. In addition, the term "medicament" as used herein encompasses a product designed for incorporation in animal feed, for example livestock feed and/or pet food.

### Dosage

The compositions of the present invention may comprise from 10⁶ to 10¹² colony forming units (CFU) of LP12418 and/or LP12407 per dose or per gram of composition, and more particularly from 10⁸ to 10¹² CFU per dose or per gram of composition. Optionally the compositions comprise about 10¹⁰ CFU LP12418 and/or LP12407 per dose or per gram of composition.

The *Lactobacillus plantarum* strains of the invention (LP12418 and/or LP12407) may be administered at a dosage of from about 10⁶ to about 10¹² CFU of bacteria per dose, preferably about 10⁸ to about 10¹² CFU of bacteria per dose. By the term "per dose" it is meant that this amount of bacteria is provided to a subject either per day or per intake, preferably per day. For example, if the bacteria are to be administered in a food product, for example in a yoghurt, then the yoghurt may contain from about 10⁶ to 10¹² CFU of LP12418 and/or LP12407. Alternatively, however, this amount of bacteria may be split into multiple administrations, each consisting of a smaller amount of microbial loading - so long as the overall amount of *Lactobacillus plantarum* received by the subject in any specific time, for instance each 24 hour period, is from about 10⁶ to about 10¹² CFU of bacteria, optionally 10⁸ to about 10¹² CFU of bacteria.

In accordance with the present invention an effective amount of LP12418 and/or LP12407 may be at least 10⁶ CFU of bacteria/dose, optionally from about 10⁸ to about 10¹² CFU of bacteria/dose, e.g., about 10¹⁰ CFU of bacteria/dose.

In one embodiment, the LP12418 and/or LP12407 may be administered at a dosage of from about 10⁶ to about 10¹² CFU of bacteria/day, optionally about 10⁸ to about 10¹² CFU of bacteria/day. Hence, the effective amount in this embodiment may be from about 10⁶ to about 10¹² CFU of bacteria/day, optionally about 10⁸ to about 10¹² CFU of bacteria/day.

### Effects/Subjects/Medical indications

The compositions of the present invention can be used for administration to a mammal, including for example livestock (including cattle, horses, pigs, and sheep), and humans. In some embodiments of the present invention, the mammal is a companion animal (including pets), such as a dog or a cat for instance. In preferred embodiments, the compositions are for use in a human.

The compositions of the present invention can be used for the prevention and/or treatment of a mental illness being a mood disorder, an anxiety disorder and/or depression, a symptom affecting mental health being anxiety, mood swings and/or depression, and/or a gastrointestinal disorder, such as irritable bowel syndrome

The term "mental illness" can be defined as a health condition that changes a person's thinking, feelings, or behaviour (or all three) and that causes the person distress and problems functioning in social, work or family activities. Mental illness encompasses a wide range of disorders related to anxiety, mood, psychosis, eating behaviour, impulse control and addiction, personality, sociability, dissociation, obsessive-compulsive and post-traumatic stress. Each illness alters a person's thoughts, feelings, and/or behaviours in distinct ways. As used herein, mental illness also includes neurological disorders and conditions related to mental illness which may be a cause or symptom of a mental illness or be a condition that can increase the chance of one developing.

Disorders associated with anxiety are categorised under "mental illness". The term "anxiety disorder" refers to a specific mental illness that involves extreme fear or worry, and includes generalized anxiety disorder (GAD), panic disorder and panic attacks, agoraphobia, social anxiety disorder, selective mutism, separation anxiety, and specific phobias. Obsessive-compulsive disorder (OCD) and posttraumatic stress disorder (PTSD) are closely related to anxiety disorders, which some may experience at the same time as depression. GAD represents more than the normal level of anxiety individuals experience from day to day and is characterised by chronic worry and tension. Compositions of the invention can be used to treat and/or prevent recognised anxiety disorders as well as symptoms of anxiety more generally.

As used herein, mental illness also includes associated neurological disorders, including memory disorders, mild cognitive impairment, dementia and Alzheimer's disease. Cognition denotes a relatively high level of processing of specific information including thinking, memory, perception, motivation, skilled movements and language. Cognitive disorders are defined as those with *"a significant impairment of cognition or memory that represents a marked deterioration from a previous level of function"* (Guerrero, Anthony (2008). Problem-Based Behavioral Science of Medicine. New York: Springer. pp. 367-79). They can be categorised into three main areas: (1) Delirium, a disorder affecting situational awareness and processing of new information; (2) Dementia, a disorder which can erase all or parts of an individual's memory; and (3) Amnesia, a disorder in which the individual afflicted has trouble retaining long term memories.

The compositions of the invention can be used to promote, restore and/or maintain an individual's mental health, such as to prevent mental illness or any associated disorders and/or symptoms affecting an individual's mental health.

Symptoms affecting mental health include; feeling sad or down, confused thinking or reduced ability to concentrate, excessive fears or worries, or extreme feelings of guilt, extreme mood changes of highs and lows, withdrawal from friends and activities, detachment from reality, paranoia or hallucinations, inability to cope with daily problems or stress, trouble understanding and relating to situations and to people, alcohol or drug abuse, major changes in eating habits, sex drive changes, excessive anger, hostility or violence and suicidal thoughts.

For the purposes of the present invention, mental illness and symptoms affecting mental health, also encompass conditions affecting an individual's cognitive function. Such conditions may include or overlap with various cognitive disorders. Examples include, but are not limited to, agnosia, amnesia, dementia, Alzheimer's disease, Parkinson's disease, and chronic stress, which has been shown to negatively affect brain function.

Intense acute and chronic stress can negatively impact both physical and mental health, increasing risk of developing mental illness. For example, chronic stress has been correlated with the development of mood disorders, anxiety disorders and depression. The compositions of the invention can be used to prevent and/or treat a mental illness or symptoms affecting mental health, resulting from chronic or acute stress.

The compositions of the invention can also be used to treat and/or prevent other (including physical) a gastrointestinal disorder, such as irritable bowel syndrome. For example, in one embodiment, the compositions of the invention are used to treat and/or prevent gastrointestinal disorders, for example, IBS, associated with chronic or acute stress. By addressing the symptoms of mental illness associated with gastrointestinal disorders, it is possible that such treatment may have a beneficial effect on the gastrointestinal disorders themselves.

More generally, the compositions of the invention can be used for the prevention and/or treatment of one or more of the mental illnesses being a mood disorder, an anxiety disorder and/or depression, symptoms affecting mental health and/or a gastrointestinal disorder, such as irritable bowel syndrome as set out above.

In particular embodiments, the compositions of the invention can be used for the prevention and/or treatment of anxiety, depression, and/or diminished cognitive function.

When compositions of the invention are used for the prevention of a mental illness being a mood disorder, an anxiety disorder and/or depression or a symptom affecting mental health being anxiety, mood swings and/or depression, they can be used for maintaining a normal level of mental health in an already healthy individual. Alternatively, when compositions of the invention are used to treat a mental illness being a mood disorder, an anxiety disorder and/or depression or symptom affecting mental health being anxiety, mood swings and/or depression, they can be used for restoring or partially restoring a normal level of mental health in an individual suffering from the mental illness or symptom in question.

### EXAMPLES

The following examples are provided in order to demonstrate and further illustrate specific embodiments and aspects of the present invention and are not to be construed as limiting the scope thereof.

### EXAMPLE 1; Initial screening of candidate strains.

Multiple probiotic candidates were initially screened for probiotic characteristics, safety and manufacturing performance. Most of these candidates were discarded due to poor performance. Efficacy testing was conducted on the remaining candidates and from these, 5 strains were selected for further experimental testing, as reported in the examples below.

Bacterial mucus adhesion properties were tested using mucus collected from five individual patients with colorectal cancer. First, the mucus was diluted with an equal volume of 0.1 M HEPES-Hank's buffer and shook vigorously for 5 min. The dilution was centrifuged once at 11,000g for 10 min (4°C) and then at 26.000g for 15 min (4°C) to remove cells and cellular debris. The supernatant was then transferred to a clean tube, and its protein content was measured using the PIERCE^{™} BCA protein assay kit. The mucus was adjusted to a protein concentration of 0.1 mg/ml using HEPES-Hank's buffer. 96-well plates were then incubated with 300 µl of mucus or the negative control bovine serum albumin (BSA) for 24 h at 4°C to coat the wells with mucus. The bacteria were grown to OD600 in MRS broth, harvested by centrifugation (8500 rpm, 15 min), and the bacterial pellet was washed once with HBSS. The bacteria were then incubated with a fluorescent Syto24 dye for 15 min on ice, centrifuged and suspended to HBSS. The coated plates were washed with HEPES-Hank's buffer to remove excess mucus or BSA, and 200 µl of bacterial suspension was added to the wells and incubated for 1 h at 37°C. The plates were again washed and the fluorescence of the adhered bacterial cells was measured. The adhesion ratio (%) was calculated by comparing the fluorescence of adhered bacterial cells to the fluorescence of unwashed cells (i.e. maximum fluorescence): Adhesion % = (Fluorescence after adhesion- Blank) / (Maximum fluorescence - Blank) x 100. The average CV% of triplicate samples varied between 15-30% depending on mucus. To determine how specifically bacteria adhered to mucus vs. other protein, an adherence ratio was calculated as: Adhesion ratio = Adhesion to mucus / Adhesion to BSA. Values above 1 indicate better affinity to mucus than BSA.

The mucus layer is the first physical barrier to host-cell stimulation by bacteria in the gut. Adhesion to this mucus is therefore the first step required for probiotic organisms to interact with host cells and elicit a response. As shown in Figure 1, the mucus adhesion test indicated that the *L. plantarum* strains LP12407 and LP12418 are superior to the other strains tested , in their ability to bind to human intestinal mucus.

### EXAMPLE 2; Materials and Methods - General

### Animals

Male Swiss mice, 5 weeks old and weighing 30-35 g, from JANVIER (Saint Berthevin, France), were used, and experiments took place at Amylgen (Direction Régionale de l'Alimentation, de l'Agriculture et de la Forêt du Languedoc-Roussillon, agreement #A 34-169-002 from May 02, 2014). Animals were housed in groups of six mice with access to food and water *ad libitum,* except during behavioural experiments. Each cage contained mice from a single treatment group. They were kept in a temperature and humidity controlled animal facility on a 12-hour light/dark cycle (lights off at 07:00 pm). All animal procedures were conducted in strict adherence to the European Union directive of September 22, 2010 (2010/63/UE).

### Bacterial strain formulation and administration

The bacterial strains were solubilized in 0.9% NaCl and administered orally by gavage (100 µL per mouse) at 9:00 a.m. each morning, corresponding to a dose of 10⁹ CFU/day per mouse. Vehicle mice received 0.9% NaCl without bacterial strains. The duration of the treatment was 33 days in total.

### Chronic stress procedure

The chronic stress procedure was carried out as previously described (Espallergues et al., Psychoneuroendocrinology, 2009). Mice were repeatedly placed in Plexiglas transparent restraint tubes (12 cm length, 3 cm diameter) under bright light for a period of 180 min per day (11:00 a.m. to 2:00 p.m.), for 5 consecutive days per week, over three weeks. Control group animals (No stress mice / Vehicle) were never placed in restraint tubes and remained undisturbed during the stress procedure in a room different from the one where stress was taking place.

### Randomisation of the animals

In each cage (N=6), animals received the same treatment. Treatments and stress procedure were performed in a random manner by an experimenter not involved in the behavioural and biochemical experiments. The behavioural procedures were conducted by a second different experimenter.

### Schedule of experimental procedures

- On day 01, animals were randomly assigned to an experimental group, weighed and treated with the appropriate strain or vehicle.
- From day 01 to day 33, animals were treated with appropriate strain or vehicle.
- From day 08 to day 28, animals were submitted to chronic stress, 3 hours per day, five days per week from Monday to Friday, in accordance with the chronic stress procedure described above.
- On day 29, animals performed the elevated-plus maze procedure.
- On day 30, animals performed the open field procedure, which was also habituation to the day 31-32 tasks.
- From day 30 to day 32, animals performed the object recognition memory task
- On day 33, animals performed the forced swim test.
- On day 36, animals were euthanized. Blood plasma samples were collected from each animal. Adrenals were collected and weighed.

During the treatment period, acute or delayed mortality was checked every day.

### Endpoint measurements

Elevated plus maze: On day 29, the anxious state of mice was measured by evaluating their ability to explore open and enclosed arms of an elevated plus-maze. The clear plexiglass apparatus consisted of two open arms (23.5 x 8 cm) and two enclosed arms (23.5 x 8 x 20 cm high), extending from a central platform and placed 50 cm above the floor. Each mouse was placed at the center of the plus-maze facing a closed arm and its exploration behaviour was recorded by Ethovision^{®} XT 9.0 (Noldus Information Technology) for 10 min. The results were expressed as locomotor activity, time spent in the open arms, and number of open arm entries. The gravity center of animal was considered by Ethovision^{®} XT 9.0 software to calculate the position of the animal in the elevated-plus maze.

Open field: On day 30, mice were placed individually in a squared open-field (50 cm x 50 cm x 50 cm high) made from white plexiglass with a floor equipped with infrared light emitting diodes. Mice were habituated to the open-field for 10 minutes and their locomotor activity captured through an IR-sensitive camera and analyzed using the Ethovision^{®} XT 9.0 software (Noldus Information Technology). Behaviour was analyzed as locomotor activity (distance traveled, cm), locomotor activity in the 25 x 25 cm central area defined by the software, and number of stereotypies (sum of the number of rearing and grooming episodes) presented by the mice.

Novel object recognition: On day 31, two identical objects (50 ml plastic vials with caps) were placed at defined positions (position #1 and position #2, at two opposite edges of the central area) of the open-field plexiglass arena. Each mouse was placed in the open-field and the exploratory activity recorded during a 10-min duration session. The activity was analyzed using the nose tracking protocol, in terms of number of contacts with objects and duration of contacts. The results are expressed as percentage of object interactions and percentage of the total interaction duration with the object in position #2.

On day 32, the object in position #2 was replaced by a novel one differing in color shape and texture from the familiar object. Each mouse was placed again in the open-field and the exploratory activity recorded during a 10- min duration session. The activity was analyzed similarly. The preferential exploration index was calculated as the ratio of the number (or duration) of contacts with the object in position #2 over the total number and duration of contacts with the two objects. Animals showing less than 10 contacts with objects during the sessions were discarded from the study.

Forced swim test: Behavioural despair, a measure of susceptibility to depression, was assessed using the forced swim test. Each mouse was placed individually in a glass cylinder (diameter 12 cm, height 24 cm) filled with water at a height of 12 cm. Water temperature was maintained at 22-23°C. The animal was forced to swim for 6 min. The session was recorded by a CCD camera connected to a computer and movements were analyzed using Ethovision^{®} XT 9.0 software (Noldus Information Technology). Two levels of pixel changes were analyzed to discriminate between immobility, struggling, and swimming. Analyses were performed min per min the last five minutes of the procedure.

Collection of brain samples: In example 3 below, brain samples are taken following euthanasia (see below). Brains were collected and dissected out on a cold plate. The hippocampus and frontal cortex were divided in two halves, dissected out and frozen on dry ice, then stored at minus 80°C. BDNF levels were measured in one half of the hippocampus.

Measurements of stress hormone release: In many species, including in rodents, corticosterone is the principle glucocorticoid involved in regulation of stress responses. In humans, cortisol is the principle glucocorticoid. Mouse plasma corticosterone levels were measured with an enzyme-linked immunosorbent assay (ELISA) from plasma samples. At sacrifice, whole blood was collected in 2 ml EDTA microtubes (ref. 061666100, Sarstedt), and immediately centrifuged at 3,000 g for 15 min in a refrigerated centrifuge at +4°C. Plasma was carefully collected and transferred to new Eppendorf tubes using a Pasteur pipette to constitute two equivalent volume aliquots. The samples were maintained at 4°C in ice during handling, then stored at minus 20°C until analysis. Plasma corticosterone was assayed with a colorimetric kit (Corticosterone (CSCI) ELISA kit, ab108821, Abcam, France) from a 25-µl sample. Mouse plasma samples were diluted 1:200 in 1X diluent M, and directly assayed according to manufacturer's instructions. The intra- and inter-assay coefficients of variation are routinely 5% and 7%, respectively. The sensitivity of the assay is routinely 0.3 ng/ml for corticosterone. Samples were assayed in duplicate. Plasma ACTH was assayed with a colorimetric kit (ACTH ELISA kit, ENZ-KIT 138, Enzo Life Science, France) in a 25-µl sample. Mouse plasma samples were diluted in 1:1 1X diluent M, and directly assayed according to manufacturer's instructions. BDNF content measurement was performed on hippocampus samples. After thawing, the hippocampal tissue was homogenized in 50mM Tris-150 mM NaCl buffer, pH 7.5, and sonicated for 20 seconds. After centrifugation, (16,100g for 15 minutes at 4°C), supernatants were used in a BDNF ELISA according to the manufacturer's instructions (Promega, #7610). Absorbance was read at 450 nm and sample concentration was calculated using the standard curve. ACTH and BDNF were both analyzed in singlets due to low sample volume, and therefore, data were analyzed for outliers with the ROUT method. Three outliers were identified for each outcome and removed from analysis.

Body weight and adrenal weight: To evaluate long-term stress hormone release, adrenals were dissected at sacrifice and weighed. Results were expressed as ratio of adrenals/body weight.

GABA receptor expression in cortex: GABA_{Aα2} and GABA_{B1β} expression was measured from frontal cortex from six mice in each group. Tissues were immersed in 1 ml of lysis reagent (QIAzol) and homogenized using high-speed shaking with a single 1 mm stainless steel bead (tissue Lyzer Qiagen, 2x2 min at 20 Hz). Chloroform was added and the sample was centrifuged to collect the RNA-containing aqueous phase. RNA was then purified using a Qiacube robotic workstation and the miRNeasy Mini kit (Qiagen). Residual DNA removal was performed using the Turbo DNase kit (Ambion), and RNA was then transcribed to cDNA using ReadyScript cDNA Synthesis Mix (Sigma-Aldrich) following the manufacturer's protocol.

Individual qPCR was performed in triplicate using SYBR Green, using ACTB as the housekeeping gene. The primer sequences were as follows: GABAAa2 forward: GGAAGCTACGCTTACACAACC, reverse: CCATCGGGAGCAACCTGAA, GABAB1b forward: CGCACCCCTCCTCAGAAC, reverse: GTCCTCCAGCGCCATCTC, ACTB forward: ATGCTCCCCGGGCTGTAT, reverse: CATAGGAGTCCTTCTGACCCATTC.

Statistical analysis: Values were expressed as mean ± Standard Error of Mean (SEM). Statistical analyses were performed using Prism 5.0a (GraphPad Software, Inc.) on the different conditions depending on results from Shapiro-Wilk normality test:
- With a one-way ANOVA (F value), followed by the Dunnett's post-hoc multiple comparison test to compare individual groups to each other, if data followed a Gaussian distribution.
- With a Kruskal-Wallis non-parametric ANOVA (H value), followed by a Dunn's multiple comparison test if data did not follow a Gaussian distribution.
*p* < 0.05 was considered to be statistically significant.

### EXAMPLE 3; Study AM306.3 Characterisation of the effect of three selected strains (LP12151, LP12407, LP12418) on repeated stress in mice

### Study protocol

Sixty Swiss mice (30 to 35g) were used in this study. Five animal groups were constituted in the following manner, according to Table 1 below.

**TABLE 1. Treatment groups in Study AM306.3**

| | n |
|---|---|
| 1. No stress male mice/Vehicle | 12 |
| 2. Chronic stressed male mice/Vehicle | 12 |
| 3. Chronic stressed male mice/ *Lactobacillus plantarum* LP12151, 10⁹ CFU/day | 12 |
| 4. Chronic stressed male mice/ *Lactobacillus plantarum* LP12407, 10⁹ CFU/day | 12 |
| 5. Chronic stress male mice/ *Lactobacillus plantarum* LP12418, 10⁹ CFU/day | 12 |
| **Total mice** | **60** |

Animals were randomly assigned to an experimental group, weighed and treated with the appropriate strain / vehicle in accordance with the methods set out in Example 2 above. From day 01 to day 33, animals were treated in accordance with the treatment schedule described in Example 2 above.

### Results and Comments

Animals weight; Figure 2 shows the effects of chronic stress on body weight in mice. Chronic stress induced a non-significant difference of body weight from day 18 to day 32 of the study as compared to the no stress/vehicle treated group. Individual treatments with the selected bacterial strains (LP12151, LP12407, LP12418) showed no effect on this parameter.

Anxiety measurement in the elevated-plus maze procedure; The effects of treatment on anxiety are illustrated in the results observed in the elevated-plus maze procedure. As seen in Figure 3, stressed mice showed a very significant anxiety-like behaviour, reflected by a reduction of the time spent in open arms (A). Treatment with LP12407 and LP12418 significantly but partially alleviated this deficit. Treatment of mice with LP12151 was ineffective at alleviating this deficit. Animal groups showed no difference in terms of locomotor activity (B) or number of open arm entries (C).

Anxiety in the open field procedure; Figure 4 shows the effects of the different treatments on chronic stress-induced anxiety in mice. As shown in Figure 4A, neither stress alone, nor any of the treatments, had an observable influence on locomotor activity. However, chronic stress did induce a very significant decrease of locomotion in the center of the arena; an indication that chronic stress induced an anxiety like behaviour (Figure 4B). Furthermore, treatment with all selected strains (LP12151, LP12407, LP12418) very significantly alleviated this deficit. Neither chronic stress alone nor any of the treatments showed any influence on the rearing/grooming behaviour, related to stereotypic activity (Figure 4C).

Recognition memory in the novel object recognition procedure; Figure 5 shows the effects of the different treatments on chronic stress-induced recognition memory in mice. Neither chronic stress alone, nor any of the treatments, showed an influence on object exploration during the session at day 2 with two identical objects, either in terms of frequency of contact (A) or in terms of time spent to explore the 2 objects (B). However, chronic stress-induced recognition memory deficits during the novel object session by decreasing the frequency of interaction with the novel object (C) and the time of interaction with the novel object (D). The selected probiotic strains (LP12151, LP12407 and LP12418) had no effect on recognition memory in this experiment.

Forced swim test; Figure 6 shows the effects of chronic stress and the various treatments on chronic stress-induced behavioural despair in mice. The forced swim test was used to measure three different parameters; immobility time (parameter A), struggle time (parameter B) and swim time (parameter C). Chronic stress induced an increase of immobility in the forced swim test (Figure 6A). Chronic stress also reduced swim time compared to the no stress/vehicle group (Figure 6C). All tested probiotic strains (LP12151, LP12407 and LP12418) fully alleviated these deficits. In terms of struggle time, neither chronic stress nor any of the various treatments appeared to influence this parameter compared to the no stress/vehicle group (Figure 6B).

Corticosterone contents measurement by ELISA; Figure 7 displays blood plasma corticosterone concentration for each of the treatment groups at day 36. Chronic stress produced a non-significant reduction in plasma corticosterone concentration in mice, that did not reach statistical significance. Treatment with LP12407 increased plasma corticosterone levels compared to the stress/vehicle and the no stress/vehicle groups. Other treatments showed no significant effects on this parameter in this study.

### General conclusions from AM306.3 study; The following conclusions can be drawn from the results described above.

Chronic stress-induced behavioural deficits:
- Induced a body weight decrease as compared to non-stressed mice
- Induced an anxiety-like state in mice in open field test and in elevated-plus maze test.
- Showed a very significant recognition long-term memory deficit in mice as compared to non-stressed mice.
- Showed a significant depressive-like state observed by behavioural despair in the forced swim test.
- Showed no significant effect on blood plasma corticosterone levels under the conditions tested.

Treatment with *L. plantarum* LP12151:
- Reduced the loss of body weight as compared to chronically stressed mice in a non-significant manner.
- Showed no effect on anxiety-like state in mice in the elevated plus maze test.
- Very significantly but partially reduced the anxiety-like state in mice in the open field test.
- Showed no effect on recognition long-term memory deficit in mice compared to chronically stressed mice.
- Showed a very significant antidepressant-like effect observed as behavioural despair in the forced swim test, compared to chronically stressed mice.
- Showed no effect on plasma corticosterone levels.

Treatment with *L. plantarum* LP12407:
- Reduced the loss of body weight as compared to chronically stressed mice in a non-significant manner.
- Very significantly but partially reduced the anxiety-like state in mice in the elevated plus maze test and in the open field test.
- Showed no effect on recognition long-term memory deficit in mice compared to chronically stressed mice.
- Showed a very significant antidepressant-like effect observed as behavioural despair in the forced swim test, compared to chronically stressed mice.
- Dramatically increased the level of corticosterone in plasma.

Treatment with *L. plantarum* LP12418:
- Reduced the loss of body weight as compared to chronically stressed mice in a non-significant manner.
- Very significantly but partially reduced the anxiety-like state in mice in the elevated plus maze test and in the open field test.
- Showed no effect on recognition long-term memory deficit in mice compared to chronically stressed mice.
- Showed a very significant antidepressant-like effect observed as behavioural despair in the forced swim test, compared to chronically stressed mice.
- Showed no effect on plasma corticosterone levels.

### EXAMPLE 4; Study AM306.5 Characterisation of the effect of three selected strains (Lpc-37, LP12418 and LP12407) on repeated stress in mice

### Study protocol

Seventy-two Swiss mice (30 to 35g) were used in this study. Five animal groups were constituted in the following manner, according to Table 2 below.

**TABLE 2. Treatment groups in Study AM306.5**

| | n | n* |
|---|---|---|
| 1. No stress male mice/Vehicle | 12 | 6* |
| 2. Chronic stressed male mice/Vehicle | 12 | 6* |
| 3. Chronic stressed male mice/ *Lactobacillus paracasei,* Lpc-37, 10⁹ CFU/day | 12 | |
| 4. Chronic stressed male mice/ *Lactobacillus plantarum,* LP12418, 10⁹ CFU/day | * | 12* |
| 5. Chronic stress male mice/ *Lactobacillus plantarum,* LP12407, 10⁹ CFU/day | 12 | |
| **Total mice** | **48** | **24** |

| | | |
|---|---|---|
| *Due to an unexpected mortality in Group 4 caused by aggressive behaviour in on cage, the 12 planned animals were discarded and this group was planned with an additional six animals per control group in a second session of experiments. | | |

Animals were randomly assigned to an experimental group, weighed and treated with the appropriate strain / vehicle. From day 01 to day 33, animals were treated in accordance with the treatment schedule described in Example 2 above. In addition, on day 28, blood was sampled and plasma prepared from the samples. Also, following euthanasia, brains were removed and the hippocampus and frontal cortex dissected out and weighed before storing at minus 80°C (also in accordance with the methods described in Example 2). Corticosterone, and ACTH levels in the plasma at day 28 were measured. BDNF content was also measured in hippocampus samples following euthanasia of mice.

### Results and Comments

Animals weight; Figure 8 shows the effects of chronic stress on body weight in mice. Chronic stress induced significant lower increase of body weight from day 18 to day 32 of the study as compared to the no stress/vehicle treated group. Individual treatments with the selected bacterial strains (Lpc-37, LP12418, LP12407) showed no effect on this parameter.

Anxiety measurement in the elevated-plus maze procedure; The effects of treatment on anxiety are illustrated in the results observed in the elevated-plus maze procedure. As seen in Figure 9, stressed mice showed a very significant anxiety-like behaviour, reflected by a reduction of the time spent in open arms (A). Treatment with Lpc-37, LP12418 or LP12407 strains very significantly but partially increased the time spent in open arms (partially alleviating the reduction observed in chronically stressed mice treated with vehicle alone). Animal groups showed no difference in terms of locomotor activity (B) or number of open arm entries (C).

Anxiety in the open field procedure; Figure 10 shows the effects of the different treatments on chronic stress-induced anxiety in mice. As shown in Figure 10A, neither stress alone, nor any of the treatments, had an observable influence on locomotor activity. However, chronic stress did induce a very significant decrease of locomotion in the center of the arena; an indication that chronic stress-induced an anxiety-like behaviour (Figure 10B). Furthermore, treatment with Lpc-37, LP12418 or LP12407 strains very significantly and fully alleviated this deficit. Chronic stress also induced a significant decrease in time spent in the inner zone(s) (D) and significantly reduced time spent in the outer zone(s) (E). Treatments with Lpc-37, LP12418 or LP12407 alleviated these stress-induced responses, restoring times spent in the inner and outer zone (s) to levels comparable to the no stress/vehicle group. Neither chronic stress alone nor any of the treatments showed any influence on the rearing/grooming behaviour, related to stereotypic activity (Figure 10C).

Recognition memory in the novel object recognition procedure; Figure 11 shows the effects of the different treatments on chronic stress-induced recognition memory in mice. Neither chronic stress alone, nor any of the treatments, showed an influence on object exploration during the session at day 2 with two identical objects, either in terms of frequency of contact (Figure 11A) or in terms of time spent to explore the 2 objects (Figure 11D). However, chronic stress-induced recognition memory deficits during the novel object session by decreasing the frequency of interaction with the novel object (Figure 11B) and the time of interaction with the novel object (Figure 11E). Treatment with either Lpc-37, LP12418 or LP12407 strains very significantly and fully restored the frequency of interaction and the time of interaction, making conclusive the effect of treatment with these strains on novel object recognition. The same profile was observed for the discrimination index (Figures 11C and 11F).

Forced swim test; Figure 12 shows the effects of chronic stress and the various treatments on chronic stress-induced behavioural despair in mice. The forced swim test was used to measure three different parameters; immobility time (parameter A), swim time (parameter B) and struggle time (parameter C). Chronic stress-induced an increase of immobility in the forced swim test (Figure 12A). Chronic stress also reduced swim time compared to the no stress/vehicle group (Figure 12B). Treatment with either Lpc-37, LP12418 or LP12407 strains very significantly and fully reversed the effects of stress on immobility time and swim time, resulting in immobility and swim time values comparable to that of the no stress/vehicle group. In terms of struggle time, neither chronic stress nor any of the various treatments appeared to influence this parameter compared to the no stress/vehicle group (Figure 12C).

Biochemical marker measurement by ELISA; Figures 13A and 13B respectively display blood plasma corticosterone and ACTH concentrations for each of the treatment groups. Figure 13C displays BDNF concentration in the hippocampus. Chronic stress very significantly increased the corticosterone concentration in mice plasma and significantly decreased the ACTH concentration. A non-significant increase in BDNF concentration was also observed in chronically stressed mice. Mice treated with Lpc-37 did not show a similar increase in blood corticosterone concentration than the chronically stressed group, although they did not significantly differ from either the non-stressed vehicle or chronically stressed vehicle mice. However, Lpc-37 treatment did not appear to affect ACTH concentration. Treatment with LP12418 showed no effect on corticosterone concentration, but very significantly increased ACTH concentration compared to the chronic stress/vehicle group, restoring ACTH concentrations to a level comparable with the no stress/ vehicle group. LP12407 treatment showed no difference in corticosterone or ACTH concentration compared to the chronically stressed mice. BDNF concentration was similar across non-stressed vehicle mice and all probiotic-treated chronically stressed mice. Despite the seemingly higher average BDNF concentration in the chronically stressed vehicle mice, there were no statistically significant differences between groups.

GABA receptor expression; Figure 14 displays the expression of two GABA receptors, GABA_{Aα2} (Figure 14A) and GABA_{B1β} (Figure 14B), in frontal cortex of mice. Chronic stress significantly reduced the expression of both receptor types compared to the no stress/vehicle group. Treatment with LP12418 significantly increased the expression of both GABA receptor types compared to stress/vehicle and completely restored expression levels to those in the no stress/vehicle group. Treatment with either Lpc-37 or LP12407 had no effect on GABA receptor expression.

### General conclusions from AM306.5 study

The following conclusions can be drawn from the results described above.

Chronic stress-induced behavioural deficits:
- Induced a partial body weight decrease as compared to non- stressed mice
- Induced an anxiety-like state in mice in open field test and in elevated-plus maze test.
- Showed a very significant recognition long-term memory deficit in mice as compared to non-stressed mice.
- Showed a significant depressive-like state observed by behavioural despair in the forced swim test paradigm
- Significantly increased blood plasma corticosterone concentration.

Treatment with either *L.paracasei* Lpc-37, *L.plantarum* LP12418, *L.plantarum,* or LP12407:
- Had no effect on the loss of body weight as compared to chronically stressed mice
- Very significantly and fully alleviated anxiety-like state in mice in the elevated-plus maze test.
- Very significantly and fully alleviated anxiety-like state in mice in the open-field procedure.
- Very significantly and fully alleviated the recognition long-term memory deficit in mice compared to chronically stressed mice.
- Very significantly and fully alleviated the depression-like behaviour observed by a behavioural despair in the forced swim test paradigm, compared to the chronically stressed group treated with vehicle alone
- Showed differential effect on corticosterone, ACTH and BDNF concentrations.
- Treatment with LP12418 restored GABA A and B receptor expression in the brain (frontal cortex), whereas Lpc-37 and LP12407 showed no effect.

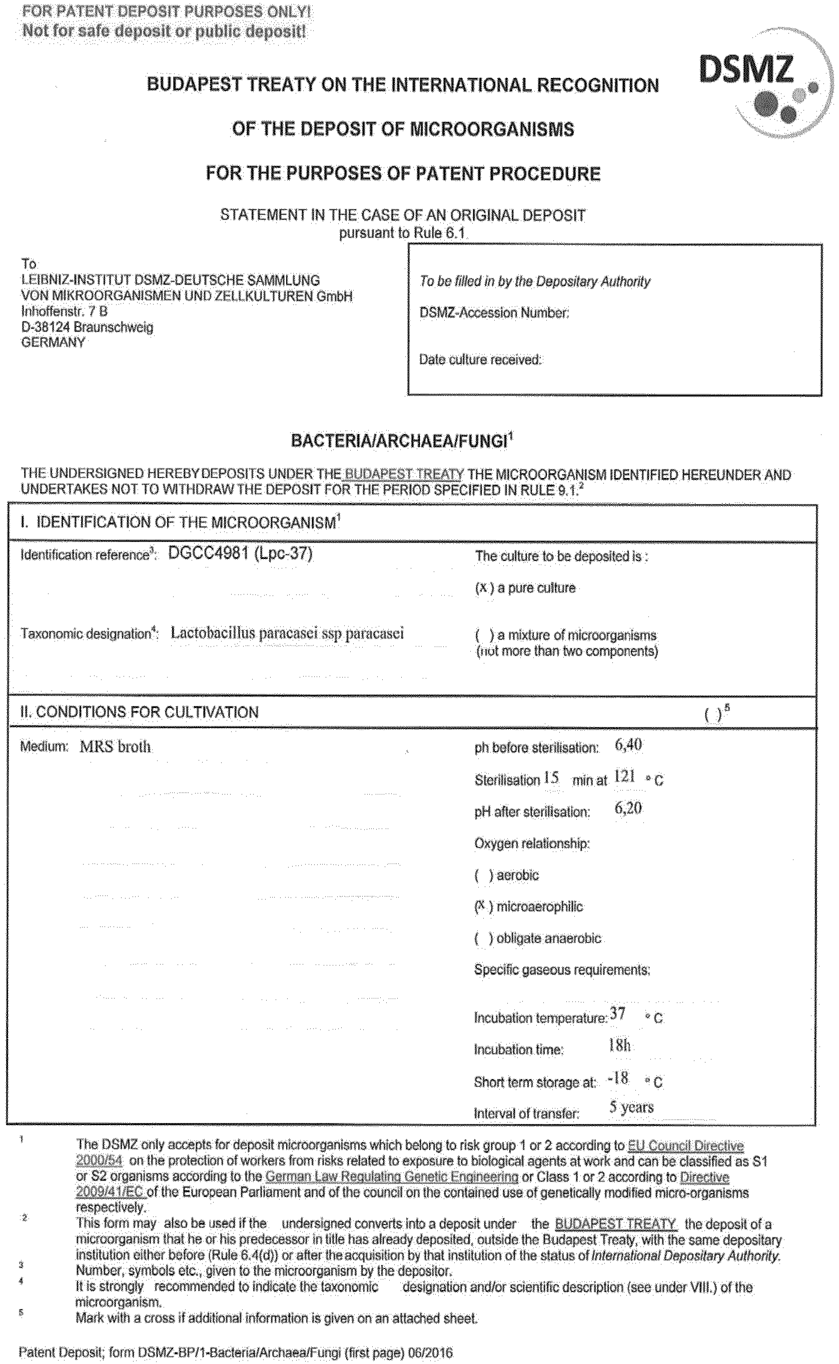
FOR PATENT DEPOSIT PURPOSES ONLY!
Not for safe deposit or public deposit!

 DSMZ

BUDAPEST TREATY ON THE INTERNATIONAL RECOGNITION

OF THE DEPOSIT OF MICROORGANISMS

FOR THE PURPOSES OF PATENT PROCEDURE

STATEMENT IN THE CASE OF AN ORIGINAL DEPOSIT
pursuant to Rule 6.1

To
LEIBNIZ-INSTITUT DSMZ-DEUTSCHE SAMMLUNG
VON MIKROORGANISMEN UND ZELLKULTUREN GmbH
Inhoffenstr. 7 B
D-38124 Braunschweig
GERMANY

To be filled in by the Depositary Authority

DSMZ-Accession Number:

Date culture received:

BACTERIA/ARCHAEA/FUNGI¹

THE UNDERSIGNED HEREBY DEPOSITS UNDER THE BUDAPEST TREATY THE MICROORGANISM IDENTIFIED HEREUNDER AND
UNDERTAKES NOT TO WITHDRAW THE DEPOSIT FOR THE PERIOD SPECIFIED IN RULE 9.1.²

I. IDENTIFICATION OF THE MICROORGANISM¹

Identification reference³: DGCC4981 (Lpc-37) | The culture to be deposited is :

(x) a pure culture

Taxonomic designation⁴: Lactobacillus paracasci ssp paracasci | ( ) a mixture of microorganisms
(not more than two components)

II. CONDITIONS FOR CULTIVATION ( )⁵

Medium: MRS broth | ph before sterilisation: 6,40

Sterilisation 15 min at 121 °C

pH after sterilisation: 6,20

Oxygen relationship:

( ) aerobic

(x) microaerophilic

( ) obligate anaerobic

Specific gaseous requirements:

Incubation temperature: 37 °C

Incubation time: 18h

Short term storage at: -18 °C

Interval of transfer: 5 years

¹ The DSMZ only accepts for deposit microorganisms which belong to risk group 1 or 2 according to EU Council Directive
2000/54 on the protection of workers from risks related to exposure to biological agents at work and can be classified as S1
or S2 organisms according to the German Law Regulating Genetic Engineering or Class 1 or 2 according to Directive
2009/41/EC of the European Parliament and of the council on the contained use of genetically modified micro-organisms
respectively.
² This form may also be used if the undersigned converts into a deposit under the BUDAPEST TREATY the deposit of a
microorganism that he or his predecessor in title has already deposited, outside the Budapest Treaty, with the same depositary
institution either before (Rule 6.4(d)) or after the acquisition by that institution of the status of International Depositary Authority.
³ Number, symbols etc., given to the microorganism by the depositor.
⁴ It is strongly recommended to indicate the taxonomic designation and/or scientific description (see under VIII.) of the
microorganism.
⁵ Mark with a cross if additional information is given on an attached sheet.

Patent Deposit; form DSMZ-BP/1-Bacteria/Archaea/Fungi (first page) 06/2016

III. CONDITIONS FOR LONG TERM STORAGE ( )⁵

propagate in MRS broth 17 to 18h at 37°C until visible growth; dilute 1:1 with fresh medium
add 10% glycerol; freeze and store below -80°C, preferably in liquid nitrogen

IV. CONDITIONS FOR TESTING VIABILITY ( )⁵

determine cell count number on MRS agar(3 days at 37°C): > 1x10E8 cfu/ml

V. COMPONENTS OF MIXED CULTURES (WHEN APPLICABLE) ( )⁵

Description of components: (not more than two components)

Method(s) for checking presence of components:

VI. PROPERTIES DANGEROUS TO HEALTH OR ENVIRONMENT ( )⁵

RISK GROUP of the microorganism¹:

(X) risk group 1 ( ) risk group 2

CLASSIFICATION in case the microorganism is genetically engineered¹ :

( ) Class 1/S1 ( ) Class 2/S2

THE STRAIN HAS TO BE HANDLED UNDER LABORATORY CONTAINMENT LEVEL¹:

(x) L1 ( ) L2

IS THIS STRAIN DANGEROUS TO HEALTH OR THE ENVIRONMENT ?

( ) YES (X) NO

if yes, please specify:

(x) the undersigned is not aware of such properties

¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.

⁵ Mark with a cross if additional information is given on an attached sheet.

Patent Deposit; form DSMZ-BP/1-Bacteria/Archaea/Fungi (second page) 06/2016

VII. IF THE MICROORGANISM IS GENETICALLY MANIPULATED ( )⁵
Please absolutely give complete answers!

1. DATA CONCERNING THE HOST ORGANISM

designation:

risk group¹: ( ) risk group 1 ( ) risk group 2

sensitivities:
resistances:
auxotrophies:

special properties:
(e.g. restriction/modification system,
general genetic recombination)

2. DATA CONCERNING THE DONOR ORGANISM

designation:

risk group¹: ( ) risk group 1 ( ) risk group 2 ( ) risk group 3

description of the cloned DNA fragment:

cloned information:

size of the cloned DNA (in bp):
( ) complete genome ( ) cDNA
( ) subgenomic ( ) subgenic ( ) synthetic

potential risk of the cloned DNA:

( ) no potential risk ( ) pathogenic ( ) tumorigenic
( ) toxigenic ( ) allergenic

3. DATA CONCERNING THE VECTOR

designation:

derivative of:

host specificity:

resistances:

plasmid size (in kb): without insert: with insert:

promoters:

additional reading frames:

own infectiosity: ( ) yes ( ) no
mobilisable plasmid: ( ) yes ( ) no
own transfer system: ( ) yes ( ) no
transfer by endogenous viruses: ( ) yes ( ) no

4. DATA CONCERNING THE GENETICALLY MANIPULATED ORGANISM

special properties:
(e.g. production of ...; use as ...-vector etc.)

foreign DNA: ( ) chromosomally integrated ( ) episomal

potential risk: ( ) pathogenic ( ) tumorigenic
( ) toxigenic ( ) allergenic
( ) no potential risk
please indicate why:

According to the regulations of the German Law Regulating Genetic Engineeringthe DSMZ can only accept genetically manipulated,
potentially pathogenic organisms for deposition when a copy of the permit issued by the competent authority (or by an equivalent
national biological safety commission) for work on the organisms accompanies the deposition form.

¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.

⁵ Mark with a cross if additional information is given on an attached sheet.

Patent Deposit; form DSMZ-BP/1-Bacteria/Archaea/Fungi (third page) 06/2016

VIII. SCIENTIFIC DESCRIPTION⁶ ( )⁵

IX. ADDITIONAL DATA⁷ ( )⁸

X. FATE OF THE CULTURE AFTER THE PRESCRIBED DURATION OF STORAGE⁹
a) The culture is to be transferred into the publicly available collection of the DSMZ | ( ) yes | ( ) no
b) The culture is to be handed back to the depositor against a fee | (X) yes | ( ) no
c) The culture is to be destroyed by the DSMZ | ( ) yes | ( ) no

XI. DEPOSITOR¹⁰
Institution/ legal entity: DuPont Nutrition Biosciences ApS
Langebrogade 1; P.O. Box 17
DK- 1001 Copenhagen K

Name of signing person(s) (typewritten):
Richard Pitman

The signing person(s) deposit(s): (X) on behalf of the legal entity
( ) as private depositor(s)

Address: Richard Pitman Signature(s):
DuPont Nutrition Biosciences ApS
Langebrogade 1; P.O. Box 17
DK- 1001 Copenhagen K

Phone: +45 32662624
Fax:
E-Mail: richard.pitman@dupont.com Date:

⁵ Mark with a cross if additional information is given on an attached sheet.
⁶ It is recommended to indicate the scientific description and/or proposed taxonomic designation (see I.) of the microorganism.
⁷ If desired name and address of the inventor(s) might be recorded here.
⁸ Mark with a cross if additional information is given on an attached sheet, such as the source of the microorganism, the name(s)
and the address(es) of anyother depositary institution(s) with which the microorganism has been deposited, or thecriterion used
when drafting the proposed taxonomic designation (The supplying of such information is optional).
⁹ The culture is to be stored for a period of at least five y ears after the most recent request for the furnishing of a sample of the
deposited organism and, in any case, for at least 30 years after the date of deposit (Rule 9.1 of the BUDAPEST TREATY). The
above regulation is valid until there will be binding jurisdiction.
¹⁰ This Deposition Form is the contract between the depositary and the depositor. Therefore it must be signed by the depositor. In
case of a legal entity the signatures of two representatives, officially nominated by this entity, are recommended. Unless otherwise
agreed, the undersigned is the correspondent of the DSMZ. Indicationof the e-mail address helps to accelerate communication.

Patent Deposit; form DSMZ-BP/1-Bacteria/Archaea/Fungi (fourth and last page) 06/2016

FOR PATENT DEPOSIT PURPOSES ONLY!
Not for safe deposit or public deposit!

DSMZ

BUDAPEST TREATY ON THE INTERNATIONAL RECOGNITION

OF THE DEPOSIT OF MICROORGANISMS

FOR THE PURPOSES OF PATENT PROCEDURE

STATEMENT IN THE CASE OF AN ORIGINAL DEPOSIT
pursuant to Rule 6.1

To
LEIBNIZ-INSTITUT DSMZ-DEUTSCHE SAMMLUNG
VON MIKROORGANISMEN UND ZELLKULTUREN GmbH
Inhoffenstr. 7 B
D-38124 Braunschweig
GERMANY

To be filled in by the Depositary Authority

DSMZ-Accession Number:

Date culture received:

BACTERIA/ARCHAEA/FUNGI¹

THE UNDERSIGNED HEREBY DEPOSITS UNDER THE BUDAPEST TREATY THE MICROORGANISM IDENTIFIED HEREUNDER AND
UNDERTAKES NOT TO WITHDRAW THE DEPOSIT FOR THE PERIOD SPECIFIED IN RULE 9.1.²

I. IDENTIFICATION OF THE MICROORGANISM¹

Identification reference³: DGCC12407 | The culture to be deposited is :

(X) a pure culture

Taxonomic designation⁴: Lactobacillus plantarum | ( ) a mixture of microorganisms
(not more than two components)

II. CONDITIONS FOR CULTIVATION | ( )⁵

Medium: MRS broth | ph before sterilisation: 6,40

Sterilisation 15 min at 121 °C

pH after sterilisation: 6,20

Oxygen relationship:

( ) aerobic

(X) microaerophilic

( ) obligate anaerobic

Specific gaseous requirements:

Incubation temperature: 37 °C

Incubation time: 18

Short term storage at: -20 °C

Interval of transfer: 5 years

¹ The DSMZ only accepts for deposit microorganisms which belong to risk group 1 or 2 according to EU Council Directive
2000/54 on the protection of workers from risks related to exposure to biological agents at work and can be classified as S1
or S2 organisms according to the German Law Regulating Genetic Engineering or Class 1 or 2 according to Directive
2009/41/EC of the European Parliament and of the council on the contained use of genetically modified micro-organisms
respectively.
² This form may also be used if the undersigned converts into a deposit under the BUDAPEST TREATY the deposit of a
microorganism that he or his predecessor in title has already deposited, outside the Budapest Treaty, with the same depositary
institution either before (Rule 6.4(d)) or after the acquisition by that institution of the status of International Depositary Authority.
³ Number, symbols etc., given to the microorganism by the depositor.
⁴ It is strongly recommended to indicate the taxonomic designation and/or scientific description (see under VIII.) of the
microorganism.
⁵ Mark with a cross if additional information is given on an attached sheet.

Patent Deposit; form DSMZ-BP/1-Bacteria/Archaea/Fungi (first page) 06/2016

III. CONDITIONS FOR LONG TERM STORAGE ( )⁵
Propagate in MRS broth 17 to 18h at 37°C until visible growth; dilute 1:1 with fresh medium
add 10% glycerol; freeze and store below -80°C, preferably in liquid nitrogen
IV. CONDITIONS FOR TESTING VIABILITY ( )⁵
determine cell count number on MRS agar (3 days at 37°C): > 1x10E8 cfu/ml
V. COMPONENTS OF MIXED CULTURES (WHEN APPLICABLE) ( )⁵
Description of components: (not more than two components)
Method(s) for checking presence of components:
VI. PROPERTIES DANGEROUS TO HEALTH OR ENVIRONMENT ( )⁵
RISK GROUP of the microorganism¹:
(X) risk group 1 ( ) risk group 2
CLASSIFICATION in case the microorganism is genetically engineered¹:
( ) Class 1/S1 ( ) Class 2/S2
THE STRAIN HAS TO BE HANDLED UNDER LABORATORY CONTAINMENT LEVEL¹:
(x) L1 ( ) L2
IS THIS STRAIN DANGEROUS TO HEALTH OR THE ENVIRONMENT ?
( ) YES (X) NO
if yes, please specify:
(x) the undersigned is not aware of such properties
¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.
⁵ Mark with a cross if additional information is given on an attached sheet.
Patent Deposit; form DSMZ-BP/1-Bacteria/Archaea/Fungi (second page) 06/2016

VII. IF THE MICROORGANISM IS GENETICALLY MANIPULATED ( )⁵
Please absolutely give complete answers!
1. DATA CONCERNING THE HOST ORGANISM
designation:
risk group¹: ( ) risk group 1 ( ) risk group 2
sensitivities:
resistances:
auxotrophies:
special properties:
(e.g. restriction/modification system,
general genetic recombination)
2. DATA CONCERNING THE DONOR ORGANISM
designation:
risk group¹: ( ) risk group 1 ( ) risk group 2 ( ) risk group 3
description of the cloned DNA fragment:
cloned information:
size of the cloned DNA (in bp):
( ) complete genome ( ) cDNA
( ) subgenomic ( ) subgenic ( ) synthetic
potential risk of the cloned DNA:
( ) no potential risk ( ) pathogenic ( ) tumorigenic
( ) toxigenic ( ) allergenic
3. DATA CONCERNING THE VECTOR
designation:
derivative of:
host specificity:
resistances:
plasmid size (in kb): without insert: with insert:
promoters:
additional reading frames:
own infectiosity: ( ) yes ( ) no
mobilisable plasmid: ( ) yes ( ) no
own transfer system: ( ) yes ( ) no
transfer by endogenous viruses: ( ) yes ( ) no
4. DATA CONCERNING THE GENETICALLY MANIPULATED ORGANISM
special properties:
(e.g. production of ...; use as ...-vector etc.)
foreign DNA: ( ) chromosomally integrated ( ) episomal
potential risk: ( ) pathogenic ( ) tumorigenic
( ) toxigenic ( ) allergenic
( ) no potential risk
please indicate why:
According to the regulations of the German Law Regulating Genetic Engineeringthe DSMZ can only accept genetically manipulated,
potentially pathogenic organisms for deposition when a copy of the permit issued by the competent authority (or by an equivalent
national biological safety commission) for work on the organisms accompanies the deposition form.
¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.
⁵ Mark with a cross if additional information is given on an attached sheet.
Patent Deposit; form DSMZ-BP/1-Bacteria/Archaea/Fungi (third page) 06/2016

VIII. SCIENTIFIC DESCRIPTION⁶ | ( )⁵

IX. ADDITIONAL DATA⁷ | ( )⁸

X. FATE OF THE CULTURE AFTER THE PRESCRIBED DURATION OF STORAGE⁹
a) The culture is to be transferred into the publicly available collection of the DSMZ | ( ) yes | ( ) no
b) The culture is to be handed back to the depositor against a fee | (X) yes | ( ) no
c) The culture is to be destroyed by the DSMZ | ( ) yes | ( ) no

XI. DEPOSITOR¹⁰
Institution/ legal entity: DuPont Nutrition Biosciences ApS
Langebrogade 1; P.O. Box 17
DK - 1001 Copenhagen K

Name of signing person(s) (typewritten):
Richard Pitman

The signing person(s) deposit(s): | (X) on behalf of the legal entity
( ) as private depositor(s)

Address: DuPont Nutrition Biosciences ApS | Signature(s):
Langebrogade 1; P.O. Box 17
DK - 1001 Copenhagen K

Phone: +45 32662624
Fax:
E-Mail: richard.pitman@dupont.com | Date:

⁵ Mark with a cross if additional information is given on an attached sheet.
⁶ It is recommended to indicate the scientific description and/or proposed taxonomic designation (see I.) of the microorganism.
⁷ If desired name and address of the inventor(s) might be recorded here.
⁸ Mark with a cross if additional information is given on an attached sheet, such as the source of the microorganism, the name(s) and the address(es) of anyother depositary institution(s) with which the microorganism has been deposited, or thecriterion used when drafting the proposed taxonomic designation (The supplying of such information is optional).
⁹ The culture is to be stored for a period of at least five y ears after the most recent request for the furnishing of a sample of the deposited organism and, in any case, for at least 30 years after the date of deposit (Rule 9.1 of the BUDAPEST TREATY). The above regulation is valid until there will be binding jurisdiction.
¹⁰ This Deposition Form is the contract between the depositary and the depositor. Therefore it must be signed by the depositor. In case of a bgal entity the signatures of two representatives, officially nominated by this entity, are recommended. Unless otherwise agreed, the undersigned is the correspondent of the DSMZ. Indicationof the e-mail address helps to accelerate communication.

Patent Deposit; form DSMZ-BP/1-Bacteria/Archaea/Fungi (fourth and last page) 06/2016

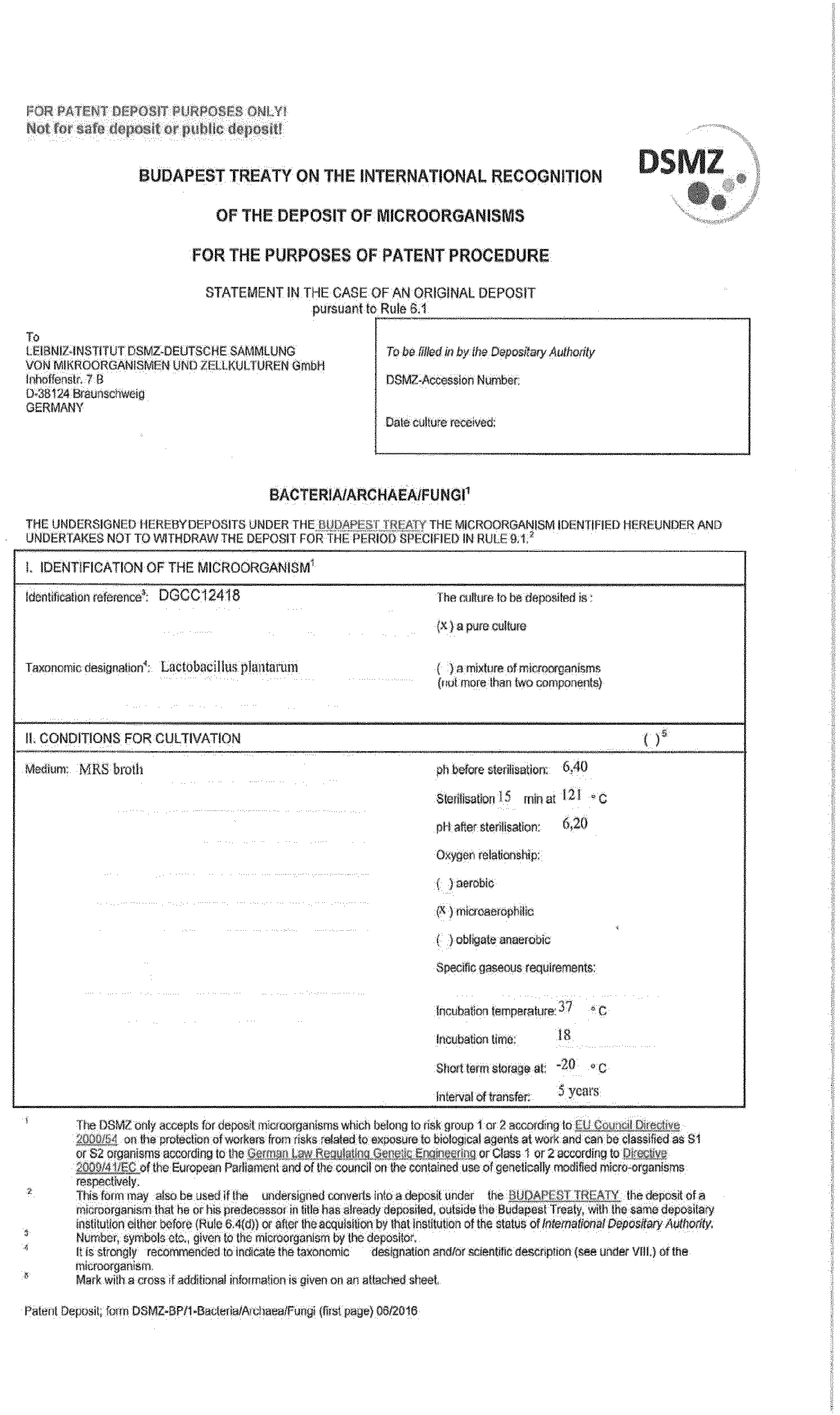
FOR PATENT DEPOSIT PURPOSES ONLY!
Not for safe deposit or public deposit!

BUDAPEST TREATY ON THE INTERNATIONAL RECOGNITION
OF THE DEPOSIT OF MICROORGANISMS
FOR THE PURPOSES OF PATENT PROCEDURE

 DSMZ

STATEMENT IN THE CASE OF AN ORIGINAL DEPOSIT
pursuant to Rule 6.1

To
LEIBNIZ-INSTITUT DSMZ-DEUTSCHE SAMMLUNG
VON MIKROORGANISMEN UND ZELLKULTUREN GmbH
Inhoffenstr. 7 B
D-38124 Braunschweig
GERMANY

To be filled in by the Depositary Authority
DSMZ-Accession Number:

Date culture received:

BACTERIA/ARCHAEA/FUNGI¹

THE UNDERSIGNED HEREBYDEPOSITS UNDER THE BUDAPEST TREATY THE MICROORGANISM IDENTIFIED HEREUNDER AND
UNDERTAKES NOT TO WITHDRAW THE DEPOSIT FOR THE PERIOD SPECIFIED IN RULE 9.1.²

I. IDENTIFICATION OF THE MICROORGANISM¹

Identification reference³: DGCC12418 | The culture to be deposited is :
(X) a pure culture
Taxonomic designation⁴: Lactobacillus plantarum | ( ) a mixture of microorganisms
(not more than two components)

II. CONDITIONS FOR CULTIVATION ( )⁵

Medium: MRS broth | ph before sterilisation: 6,40
Sterilisation 15 min at 121 °C
pH after sterilisation: 6,20
Oxygen relationship:
( ) aerobic
(X) microaerophilic
( ) obligate anaerobic
Specific gaseous requirements:

Incubation temperature: 37 °C
Incubation time: 18
Short term storage at: -20 °C
Interval of transfer: 5 years

¹ The DSMZ only accepts for deposit microorganisms which belong to risk group 1 or 2 according to EU Council Directive
2000/54 on the protection of workers from risks related to exposure to biological agents at work and can be classified as S1
or S2 organisms according to the German Law Regulating Genetic Engineering or Class 1 or 2 according to Directive
2009/41/EC of the European Parliament and of the council on the contained use of genetically modified micro-organisms
respectively.
² This form may also be used if the undersigned converts into a deposit under the BUDAPEST TREATY the deposit of a
microorganism that he or his predecessor in title has already deposited, outside the Budapest Treaty, with the same depositary
institution either before (Rule 6.4(d)) or after the acquisition by that institution of the status of International Depositary Authority.
³ Number, symbols etc., given to the microorganism by the depositor.
⁴ It is strongly recommended to indicate the taxonomic designation and/or scientific description (see under VIII.) of the
microorganism.
⁵ Mark with a cross if additional information is given on an attached sheet.

Patent Deposit; form DSMZ-BP/1-Bacteria/Archaea/Fungi (first page) 06/2016

III. CONDITIONS FOR LONG TERM STORAGE | ( )⁵
Propagate in MRS broth 17 to 18h at 37°C until visible growth; dilute 1:1 with fresh medium
add 10% glycerol; freeze and store below -80°C, preferably in liquid nitrogen
IV. CONDITIONS FOR TESTING VIABILITY | ( )⁵
determine cell count number on MRS agar (3 days at 37°C): > 1x10E8 cfu/ml
V. COMPONENTS OF MIXED CULTURES (WHEN APPLICABLE) | ( )⁵
Description of components: (not more than two components)
Method(s) for checking presence of components:
VI. PROPERTIES DANGEROUS TO HEALTH OR ENVIRONMENT | ( )⁵
RISK GROUP of the microorganism¹:
(X) risk group 1 | ( ) risk group 2
CLASSIFICATION in case the microorganism is genetically engineered¹:
( ) Class 1/S1 | ( ) Class 2/S2
THE STRAIN HAS TO BE HANDLED UNDER LABORATORY CONTAINMENT LEVEL¹:
(x) L1 | ( ) L2
IS THIS STRAIN DANGEROUS TO HEALTH OR THE ENVIRONMENT ?
( ) YES | (X) NO
if yes, please specify:
(x) the undersigned is not aware of such properties
¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.
⁵ Mark with a cross if additional information is given on an attached sheet.
Patent Deposit; form DSMZ-BP/1-Bacteria/Archaea/Fungi (second page) 06/2016

VII. IF THE MICROORGANISM IS GENETICALLY MANIPULATED ( )⁵
Please absolutely give complete answers!

1. DATA CONCERNING THE HOST ORGANISM

designation:

risk group¹: ( ) risk group 1 ( ) risk group 2

sensitivities:
resistances:
auxotrophies:

special properties:
(e.g. restriction/modification system,
general genetic recombination)

2. DATA CONCERNING THE DONOR ORGANISM

designation:

risk group¹: ( ) risk group 1 ( ) risk group 2 ( ) risk group 3

description of the cloned DNA fragment:

cloned information:

size of the cloned DNA (in bp):
( ) complete genome ( ) cDNA
( ) subgenomic ( ) subgenic ( ) synthetic

potential risk of the cloned DNA:

( ) no potential risk ( ) pathogenic ( ) tumorigenic
( ) toxigenic ( ) allergenic

3. DATA CONCERNING THE VECTOR

designation:

derivative of:

host specificity:

resistances:

plasmid size (in kb): without insert: with insert:

promoters:

additional reading frames:

own infectiosity: ( ) yes ( ) no
mobilisable plasmid: ( ) yes ( ) no
own transfer system: ( ) yes ( ) no
transfer by endogenous viruses: ( ) yes ( ) no

4. DATA CONCERNING THE GENETICALLY MANIPULATED ORGANISM

special properties:
(e.g. production of ...; use as ...-vector etc.)

foreign DNA: ( ) chromosomally integrated ( ) episomal

potential risk: ( ) pathogenic ( ) tumorigenic
( ) toxigenic ( ) allergenic
( ) no potential risk
please indicate why:

According to the regulations of the German Law Regulating Genetic Engineeringthe DSMZ can only accept genetically manipulated,
potentially pathogenic organisms for deposition when a copy of the permit issued by the competent authority (or by an equivalent
national biological safety commission) for work on the organisms accompanies the deposition form.

¹ The DSMZ only accepts for deposit microorganisms which belong to risk group 1 or 2 according to EU Council Directive
2000/54 on the protection of workers from risks related to exposure to biological agents at work and can be classified as S1 or
S2 organisms according to the German Law Regulating Genetic Engineering or Class 1 or 2 according to Directive
2009/41/EC of the European Parliament and of the council on the contained use of genetically modified micro-organisms
respectively.

⁵ Mark with a cross if additional information is given on an attached sheet.

Patent Deposit; form DSMZ-BP/1-Bacteria/Archaea/Fungi (third page) 06/2016

VIII. SCIENTIFIC DESCRIPTION⁶ | ( )⁵

IX. ADDITIONAL DATA⁷ | ( )⁸

X. FATE OF THE CULTURE AFTER THE PRESCRIBED DURATION OF STORAGE⁹
a) The culture is to be transferred into the publicly available collection of the DSMZ | ( ) yes | ( ) no
b) The culture is to be handed back to the depositor against a fee | (X) yes | ( ) no
c) The culture is to be destroyed by the DSMZ | ( ) yes | ( ) no

XI. DEPOSITOR¹⁰
Institution/ legal entity: DuPont Nutrition Biosciences ApS
Langebrogade 1; P.O. Box 17
DK - 1001 Copenhagen K

Name of signing person(s) (typewritten):
Richard Pitman

The signing person(s) deposit(s): | (X) on behalf of the legal entity
( ) as private depositor(s)

Address: DuPont Nutrition Biosciences ApS | Signature(s):
Langebrogade 1; P.O. Box 17
DK - 1001 Copenhagen K

Phone: +45 32662624
Fax:
E-Mail: richard.pitman@dupont.com | Date:

⁵ Mark with a cross if additional information is given on an attached sheet.
⁶ It is recommended to indicate the scientific description and/or proposed taxonomic designation (see I.) of the microorganism.
⁷ If desired name and address of the inventor(s) might be recorded here.
⁸ Mark with a cross if additional information is given on an attached sheet, such as the source of the microorganism, the name(s) and the address(es) of anyother depositary institution(s) with which the microorganism has been deposited, or thecriterion used when drafting the proposed taxonomic designation (The supplying of such information is optional).
⁹ The culture is to be stored for a period of at least five y ears after the most recent request for the furnishing of a sample of the deposited organism and, in any case, for at least 30 years after the date of deposit (Rule 9.1 of the BUDAPEST TREATY). The above regulation is valid until there will be binding jurisdiction.
¹⁰ This Deposition Form is the contract between the depositary and the depositor. Therefore it must be signed bythe depositor. In case of a legal entity the signatures of two representatives, officially nominated by this entity, are recommended. Unless otherwise agreed, the undersigned is the correspondent of the DSMZ. Indicationof the e-mail address helps to accelerate communication.

Patent Deposit; form DSMZ-BP/1-Bacteria/Archaea/Fungi (fourth and last page) 06/2016

## Claims

1. A bacterial strain of the species *Lactobacillus plantarum,* wherein the bacterial strain is strain LP12418 deposited with the DSMZ under deposit number DSM 32655 on September 27, 2017.

2. A bacterial strain of the species *Lactobacillus plantarum,* wherein the bacterial strain is strain LP12407 deposited with the DSMZ under deposit number DSM 32654 on September 27, 2017.

3. Bacteria of the species *Lactobacillus plantarum* or a mixture thereof for use as a medicament, wherein the bacteria of the species *Lactobacillus plantarum* is strain LP12418 deposited with the DSMZ under deposit number DSM 32655 on September 27, 2017 and/or strain LP12407 deposited with the DSMZ under deposit number DSM 32654 on September 27, 2017.

4. A composition comprising *Lactobacillus plantarum* strain LP12418 deposited with the DSMZ under deposit number DSM 32655 on September 27, 2017.

5. A composition comprising *Lactobacillus plantarum* strain LP12407 deposited with the DSMZ under deposit number DSM 32654 on September 27, 2017.

6. A composition according to claim 4 or claim 5, wherein the composition further comprises another bacterial strain.

7. A composition according to claim 6, wherein the other bacterial strain is of the species, *Lactobacillus paracasei,* e.g. strain Lpc-37, deposited with the DSMZ under deposit number DSM 32661 on October 5, 2017.

8. A composition comprising *Lactobacillus plantarum* strain LP12418 deposited with the DSMZ under deposit number DSM 32655 on September 27, 2017, *Lactobacillus plantarum* strain LP12407 deposited with the DSMZ under deposit number DSM 32654 on September 27, 2017, and *Lactobacillus paracasei* strain Lpc-37, deposited with the DSMZ under deposit number DSM 32661 on October 5, 2017.

9. A composition according to any of claims 4 to 8, wherein the composition is in the form of a food product, a beverage, a dietary supplement or a pharmaceutically acceptable composition.

10. A composition according to any of claims 4 to 9, wherein the composition is a spray dried or freeze-dried composition.

11. A composition according to claim 10, wherein the composition comprises a cryoprotectant.

12. A composition according to any of claims 4 to 7, wherein the composition is in unit dosage form and wherein the *Lactobacillus plantarum* strain is present in the composition in an amount between 10⁶ and 10¹² CFU, e.g. between 10⁸ and 10¹² CFU, optionally 10¹⁰ CFU per unit dose.

13. A composition according to any of claims 4 to 12, for use as a medicament.

14. A composition according to claim 13 for use in preventing and/or treating a mood disorder, mood swings, an anxiety disorder and/or depression, diminished cognitive function and/or a gastrointestinal disorder, such as irritable bowel syndrome, in a mammal.
the symptom affecting mental health is anxiety, mood swings and/or depression

## Patentansprüche

1. Bakterieller Stamm der Spezies *Lactobacillus plantarum,* wobei es sich bei dem bakteriellen Stamm um Stamm LP12418 handelt, der bei der DSMZ unter Hinterlegungsnummer DSM 32655 am 27. September 2017 hinterlegt wurde.

2. Bakterieller Stamm der Spezies *Lactobacillus plantarum,* wobei es sich bei dem bakteriellen Stamm um Stamm LP12407 handelt, der bei der DSMZ unter Hinterlegungsnummer DSM 32654 am 10. 27. September 2017 hinterlegt wurde.

3. Bakterien der Spezies *Lactobacillus plantarum* oder Mischung davon zur Verwendung als Medikament, wobei die Bakterien der Spezies *Lactobacillus plantarum* dem Stamm LP12418, der bei der DSMZ unter Hinterlegungsnummer DSM 32655 am 27. September 2017 hinterlegt wurde, und/oder dem Stamm LP12407, der bei der DSMZ unter Hinterlegungsnummer DSM 32654 am 27. September 2017 hinterlegt wurde, zugehörig sind.

4. Zusammensetzung, umfassend *Lactobacillus plantarum-*Stamm LP12418, der bei der DSMZ unter Hinterlegungsnummer DSM 32655 am 27. September 2017 hinterlegt wurde.

5. Zusammensetzung, umfassend *Lactobacillus plantarum-*Stamm LP12407, der bei der DSMZ unter Hinterlegungsnummer DSM 32654 am 27. September 2017 hinterlegt wurde.

6. Zusammensetzung nach Anspruch 4 oder Anspruch 5, wobei die Zusammensetzung ferner einen anderen bakteriellen Stamm umfasst.

7. Zusammensetzung nach Anspruch 6, wobei der andere bakterielle Stamm der Spezies *Lactobacillus paracasei* zugehört, z. B. Stamm Lpc-37, der bei der DSMZ unter Hinterlegungsnummer DSM 32661 am 5. Oktober 2017 hinterlegt wurde.

8. Zusammensetzung, umfassend *Lactobacillus plantarum-*Stamm LP12418, der bei der DSMZ unter der Hinterlegungsnummer DSM 32655 am 27. September 2017 hinterlegt wurde, *Lactobacillus plantarum-Stamm* LP12407, der bei der DSMZ unter der Hinterlegungsnummer DSM 32654 am 27. September 2017 hinterlegt wurde und *Lactobacillus paracasei-Stamm* Lpc-37, der bei der DSMZ unter der Hinterlegungsnummer DSM 32661 am 5. Oktober 2017 hinterlegt wurde.

9. Zusammensetzung nach einem der Ansprüche 4 bis 8, wobei die Zusammensetzung in Form eines Lebensmittels, eines Getränks, eines Nahrungsergänzungsmittels oder einer pharmazeutisch unbedenklichen Zusammensetzung vorliegt.

10. Zusammensetzung nach einem der Ansprüche 4 bis 9, wobei die Zusammensetzung einer Sprühtrocknung oder Gefriertrocknung unterzogen wurde.

11. Zusammensetzung nach Anspruch 10, wobei die Zusammensetzung ein Kryoprotektivum umfasst.

12. Zusammensetzung nach einem der Ansprüche 4 bis 7, wobei die Zusammensetzung in einer Einheitsdarreichungsform vorliegt und wobei der *Lactobacillus plantarum-Stamm* in der Zusammensetzung in einer Menge zwischen 10⁶ und 10¹² KBE, z. B. zwischen 10⁸ und 10¹² KBE, gegebenenfalls 10¹⁰ KBE pro Einheitsdosis vorhanden ist.

13. Zusammensetzung nach einem der Ansprüche 4 bis 12 zur Verwendung als Medikament.

14. Zusammensetzung nach Anspruch 13 zur Verwendung beim Vorbeugen und/oder Behandeln einer affektiven Störung, von Stimmungsschwankungen, einer Angststörung und/oder Depression, von verminderter kognitiver Funktion und/oder einer Magen-Darm-Erkrankung, wie Reizdarmsyndrom, bei einem Säugetier.

## Revendications

1. Souche bactérienne de l'espèce *Lactobacillus plantarum,* la souche bactérienne étant la souche LP12418 ayant fait l'objet d'un dépôt auprès du DSMZ sous le numéro de dépôt DSM 32655 le 27 septembre 2017.

2. Souche bactérienne de l'espèce *Lactobacillus plantarum,* la souche bactérienne étant la souche LP12407 ayant fait l'objet d'un dépôt auprès du DSMZ sous le numéro de dépôt DSM 32654 le 10 27 septembre 2017.

3. Bactéries de l'espèce *Lactobacillus plantarum,* ou mélange de celles-ci, destinées à être utilisées comme médicament, les bactéries de l'espèce *Lactobacillus plantarum* étant la souche LP12418 ayant fait l'objet d'un dépôt auprès du DSMZ sous le numéro de dépôt DSM 32655 le 27 septembre 2017 et/ou la souche LP12407 ayant fait l'objet d'un dépôt auprès du DSMZ sous le numéro de dépôt DSM 32654 le 27 septembre 2017.

4. Composition comprenant la souche LP12418 de *Lactobacillus plantarum* ayant fait l'objet d'un dépôt auprès du DSMZ sous le numéro de dépôt DSM 32655 le 27 septembre 2017.

5. Composition comprenant la souche LP12407 de *Lactobacillus plantarum* ayant fait l'objet d'un dépôt auprès du DSMZ sous le numéro de dépôt DSM 32654 le 27 septembre 2017.

6. Composition selon la revendication 4 ou la revendication 5, la composition comprenant en outre une autre souche bactérienne.

7. Composition selon la revendication 6, dans laquelle l'autre souche bactérienne est de l'espèce *Lactobacillus paracasei,* à titre d'exemple la souche Lpc-37 ayant fait l'objet d'un dépôt auprès du DSMZ sous le numéro de dépôt DSM 32661 le 05 octobre 2017.

8. Composition comprenant la souche LP12418 de *Lactobacillus plantarum* ayant fait l'objet d'un dépôt auprès du DSMZ sous le numéro de dépôt DSM 32655 le 27 septembre 2017, la souche LP12407 de *Lactobacillus plantarum* ayant fait l'objet d'un dépôt auprès du DSMZ sous le numéro de dépôt DSM 32654 le 27 septembre 2017 et la souche Lpc-37 de *Lactobacillus paracasei* ayant fait l'objet d'un dépôt auprès du DSMZ sous le numéro de dépôt DSM 32661 le 05 octobre 2017.

9. Composition selon l'une quelconque des revendications 4 à 8, la composition étant sous forme d'un produit alimentaire, d'une boisson, d'un complément alimentaire ou d'une composition acceptable d'un point de vue pharmaceutique.

10. Composition selon l'une quelconque des revendications 4 à 9, la composition étant une composition séchée par pulvérisation ou lyophilisée.

11. Composition selon la revendication 10, la composition comprenant un cryo-protecteur.

12. Composition selon l'une quelconque des revendications 4 à 7, la composition étant dans une forme posologique unitaire et dans laquelle la souche de *Lactobacillus plantarum* est présente dans la composition en une quantité comprise entre 10⁶ et 10¹² CFU, à titre d'exemple entre 10⁸ et 10¹² CFU, en option 10¹⁰ CFU par dose unitaire.

13. Composition selon l'une quelconque des revendications 4 à 12, destinée à être utilisée comme médicament.

14. Composition, selon la revendication 13, destinée à être utilisée dans la prévention et/ou le traitement d'un trouble de l'humeur, de sauts d'humeur, d'un trouble anxieux et/ou de la dépression, d'une fonction cognitive diminuée et/ou d'un trouble gastro-intestinal, tel que le syndrome du côlon irritable, chez un mammifère.
